# EUROPEAN PATENT SPECIFICATION

(11) **EP 1 589 949 B1**
(45) Date of publication and mention of the grant of the patent: **13.08.2008**
(21) Application number: 04700827.1
(22) Date of filing: 08.01.2004
(51) Int. Cl.: A61K 9/19

(54) **STABILIZED LYOPHILIZED COMPOSITIONS COMPRISING TISSUE FACTOR PATHWAY INHIBITOR OR TISSUE FACTOR PATHWAY INHIBITOR VARIANTS**
STABILISIERTE LYOPHILISIERTE ZUBEREITUNGEN MIT GEWEBEFAKTOR-INHIBITOR (TFPI) ODER VARIANTEN DES GEWEBEFAKTOR-INHIBITORS
COMPOSITIONS LYOPHILISEES STABILISEES RENFERMANT UN INHIBITEUR DE LA VOIE DU FACTEUR TISSULAIRE OU DES VARIANTES D'UN INHIBITEUR DE LA VOIE DU FACTEUR TISSULAIRE

(30) Priority: 08.01.2003 US 438524 P; 13.08.2003 US 494547 P; 08.10.2003 US 509276 P; 20.10.2003 US 512092 P
(43) Date of publication of application: 02.11.2005
(60) Divisional application: 06077319.9
(73) Proprietor: Novartis Vaccines and Diagnostics, Inc., Emeryville, CA 94608 (US)
(72) Inventor: CHEN, Bao-lu, San Ramon, CA 94583 (US); HORA, Maninder, Danville, CA 94596 (US)
(74) Representative: Marshall, Cameron John
(86) International application number: PCT/US2004/000235
(87) International publication number: WO 2004/062646

(56) References cited:
- WO-A-01/24814
- WO-A-01/49274
- WO-A-98/33920
- US-A- 5 888 968

## Description

### FIELD OF THE INVENTION

The invention relates generally to the field of stabilized formulation of proteins. More specifically, the invention relates to stabilized lyophilized compositions of tissue factor pathway inhibitor (TFPI) and TFPI variants.

### BACKGROUND OF THE INVENTION

Tissue factor pathway inhibitor (TFPI) is 276 amino acids in length and functions as an inhibitor of tissue factor-mediated blood coagulation. Its amino acid sequence is shown in SEQ ID NO:1. The amino terminal end of TFPI is negatively charged, and the carboxy terminal end is positively charged. The TFPI protein contains three Kunitz-type enzyme inhibitor domains. TFPI contains 18 cysteine residues and forms 9 disulfide bridges when correctly folded. The primary sequence contains three N-linked consensus glycosylation sites (Asn-X-Ser/Thr). The asparagine residues of the glycosylation sites are located at positions 145, 195 and 256. TFPI is also known as lipoprotein associated coagulation inhibitor (LACI), tissue factor inhibitor (TFI), and extrinsic pathway inhibitor (EPI).

Use of TFPI has been proposed for the treatment of various indications, including sepsis (U.S. 6,063,764 and WO 93/24143), deep vein thrombosis (U.S. 5,563,123, U.S. 5,589,359, and WO 96/04378), ischemia (U.S. 5,885,781, U.S. 6,242,414, and WO 96/40224), restenosis (U.S. 5,824,644 and WO 96/01649), and cancer (U.S. 5,902,582 and WO 97/09063). A TFPI variant, which differs from TFPI by the addition of an alanine residue at the amino terminus ("ala-TFPI"), has been shown to be efficacious in animal models for the treatment of sepsis. Carr et al., Circ Shock 1994 Nov;44(3):126-37.

TFPI is a hydrophobic protein with limited solubility in aqueous solutions. Aggregation of TFPI in solution has been correlated with loss of biological activity. Various formulations have been made; see, for example, U.S 5,888,968 and WO 96/40784. There is, however, a continuing need in the art for stabilized compositions of TFPI or TFPI variants. 17

### BRIEF SUMMARY OF THE INVENTION

The invention provides a lyophilized composition according to the claims. The lyophilized composition of TFPI or TFPI variant comprises (1) TFPI or TFPI variant and (2) a carbohydrate or amino acid glass forming agent. The lyophilized composition may have about 45% or greater aggregation stability.

Before lyophilization the TFPI or TFPI variant may be present in an aqueous formulation comprising a carbohydrate or amino acid glass forming agent, wherein the aqueous formulation has a pH of about 4 to about 8.

A lyophilized composition of TFPI or TFPI variant may be prepared by the following method. The method comprises the step of lyophilizing an aqueous formulation comprising (1) TFPI or TFPI variant and (2) a carbohydrate or amino acid glass forming agent, wherein the aqueous formulation has a pH of about 4 to about 8, whereby a lyophilized composition of the TFPI or TFPI variant having about 45% or greater aggregation stability is formed.

The preparation of a composition of

TFPI or TFPI variant for lyophilization may be aided by the following process. The process comprises the step of removing a chaotrope from a first formulation comprising (1) TFPI or TFPI variant and (2) the chaotrope to form a second formulation that comprises the TFPI or TFPI variant and is essentially free of the chaotrope, wherein the second formulation has a pH of about 3.5 to about 4.5. 19. The chaotrope can be urea. In some embodiments, the second formulation has a pH of about 4. The first formulation can comprise about 300 mM arginine and about 20 mM sodium citrate with a pH of about 5.5; about 2M urea, about 150 mM sodium chloride, and about 20 mM sodium phosphate with a pH of about 7.2; about 2M urea, about 250 mM sodium chloride, and about 20 mM sodium phosphate with a pH of about 7.2; or about 1M urea, about 125 mM sodium chloride, and about 10 mM sodium phosphate with a pH of about 7. The second formulation can comprise about 300 mM arginine and about 20 mM sodium citrate with a pH of about 5.5; about 1% (w/v) sucrose, about 4% (w/v) mannitol, about 10 mM histidine with a pH of about 6; about 1% (w/v) sucrose, about 4% (w/v) mannitol, and about 10 mM glutamate with a pH of about 4; or about 3% (w/v) arginine, about 4% (w/v) mannitol, and about 10 mM histidine with a pH of about 6. The step of removing can be performed by at least one method selected from the group consisting of diafiltration, dialysis, and size exclusion chromatography.

The preparation of a composition of TFPI or TFPI variant for lyophilization may be aided by the following process. The process comprises the step of replacing a first formulation low molecular weight solute in a first formulation comprising (1) TFPI or TFPI variant and (2) the first formulation low molecular weight solute with a second solution low molecular weight solute to form a second formulation, wherein the second formulation has a pH of about 3.5 to about 4.5. The process can comprise the step of replacing second formulation solute with a third formulation solute to form a third formulation. The step of replacing can be performed by diafiltration. The third formulation can be a pharmaceutically acceptable formulation. The step of replacing the second formulation solute can be performed by a method selected from the group consisting of diafiltration, dialysis, and size exclusion chromatography. If desired, the third formulation can comprise about 1% (w/v) sucrose, about 4% (w/v) mannitol, and about 10 mM histidine with a pH of about 6; and about 1% (w/v) sucrose, about 4% (w/v) mannitol, and about 10 mM imidazole with a pH of about 6.5.

The lyophilized composition of TFPI or TFPI variant may comprise (1) TFPI or TFPI variant and (2) a citrate buffer, wherein the lyophilized composition has about 45% or greater aggregation stability.

The lyophilized composition of TFPI or TFPI variant may comprise (1) TFPI or TFPI variant, (2) sulfate, and (3) a phosphate buffer, wherein the lyophilized composition has about 45% or greater aggregation stability.

A lyophilized composition of TFPI or TFPI variant may be prepared by the following method. The method comprises the step of lyophilizing an aqueous formulation selected from the group consisting of TFPI or TFPI variant and a citrate buffer; and TFPI or TFPI variant, sulfate, and a phosphate buffer, whereby a lyophilized composition of the TFPI or TFPI variant having about 45% or greater aggregation stability is formed.

### BRIEF DESCRIPTION OF THE DRAWINGS

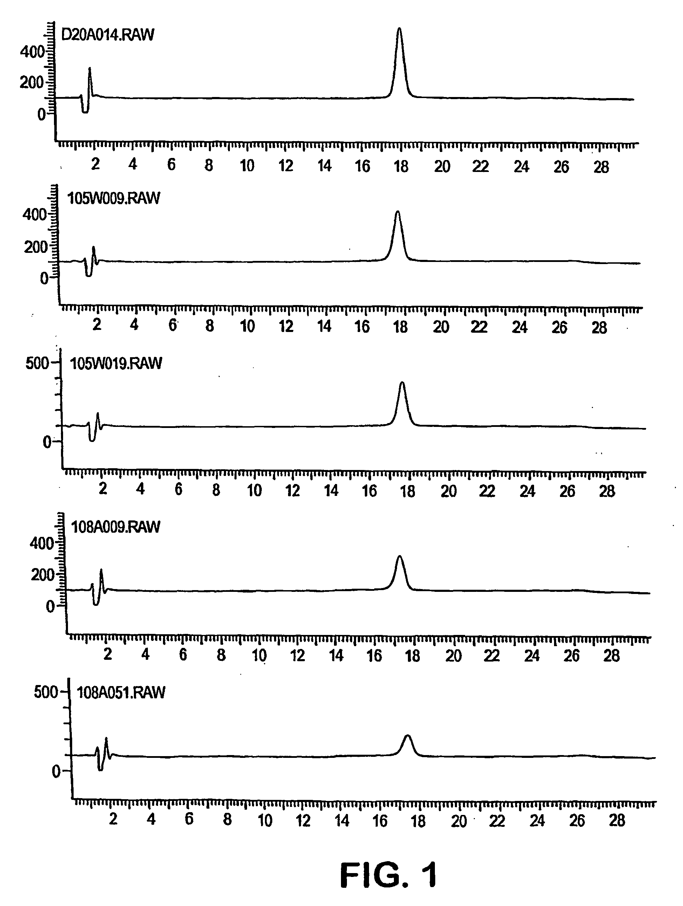

FIG. 1 shows chromatograms of cation exchange high performance liquid chromatography of ala-TFPI for testing stability of samples prepared in aqueous formulation at pH 7. The aqueous formulations contained 150 µg/ml ala-TFPI, 10 mM sodium phosphate at pH 7, 150 mM NaCI and 0.015% (w/v) polysorbate-80. The aqueous formulations were stored at 40°C for 0, 3, 5, 10, or 20 days (from top to bottom, respectively).

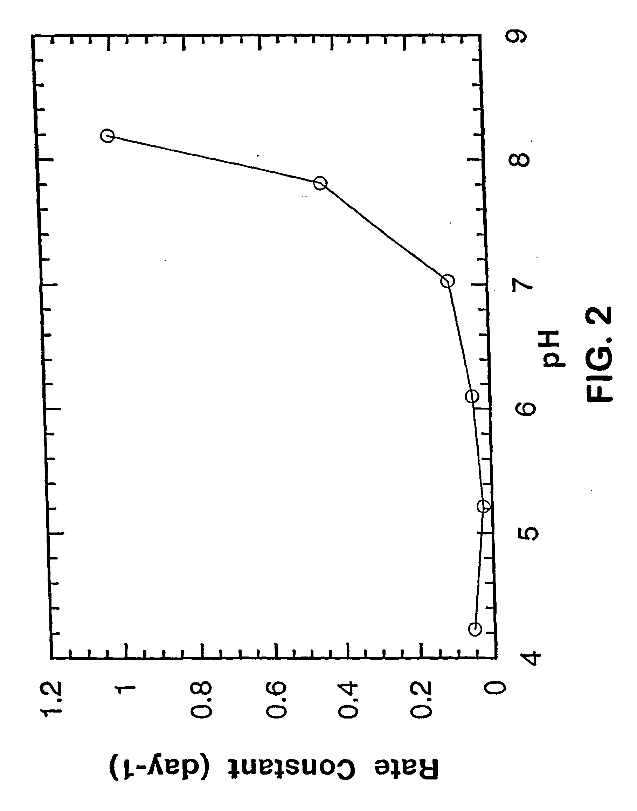

FIG. 2 shows the first order rate constant for loss of soluble ala-TFPI as a function of pH at 40°C in aqueous formulations. The aqueous formulations contained 150 µg/ml ala-TFPI, 10 mM sodium phosphate, 150 mM NaCl and 0.015% (w/v) polysorbate-80.

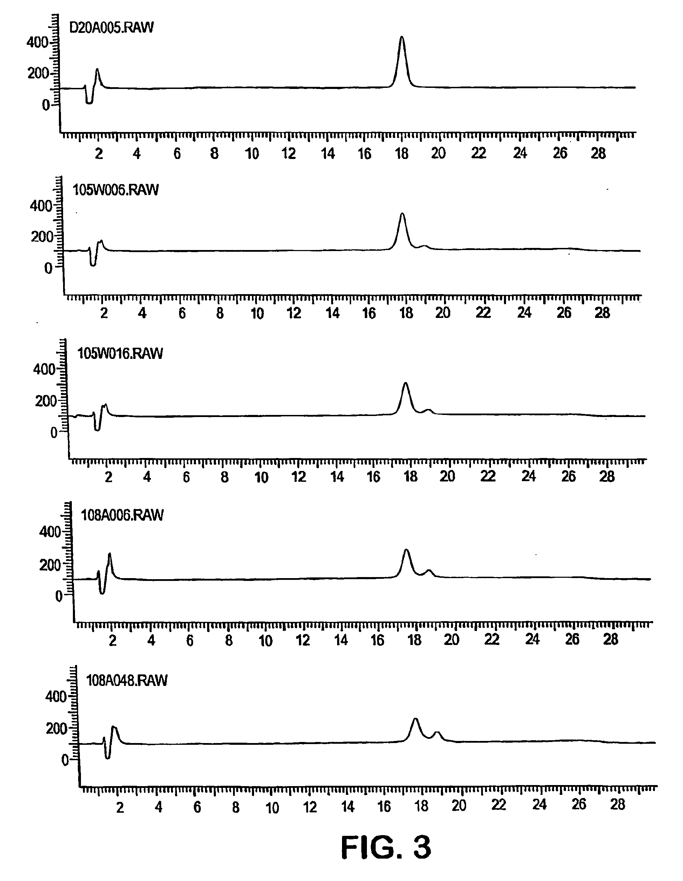

FIG. 3 shows chromatograms of cation exchange high performance liquid chromatography of ala-TFPI for testing stability of samples prepared in aqueous formulation at pH 4. The aqueous formulations contained 150 µg/ml ala-TFPI, 10 mM sodium phosphate at pH 4, 150 mM NaCl and 0.015% (w/v) polysorbate-80. The aqueous formulations were stored at 40°C for 0, 3, 5, 10, or 20 days (from top to bottom, respectively).

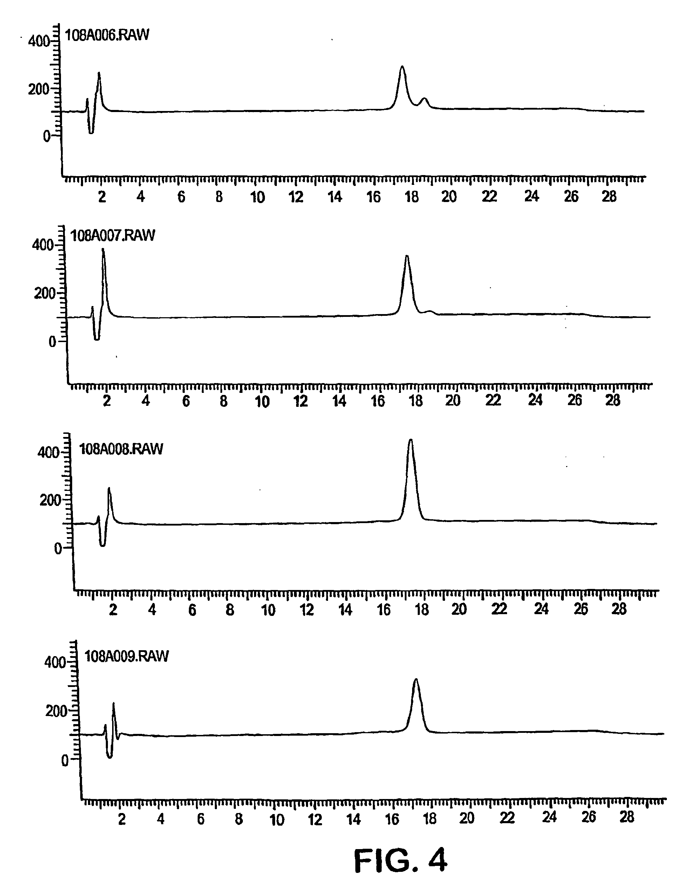

FIG. 4 shows chromatograms of cation exchange high performance liquid chromatography of ala-TFPI for testing stability of samples prepared in aqueous formulation at various pH values. The aqueous formulations contained 150 µg/ml ala-TFPI, 10 mM sodium phosphate, 150 mM NaCl and 0.015% (w/v) polysorbate-80. The aqueous formulations were stored at 40°C for 10 days. The pH values are pH 4, 5, 6 or 7 (from top to bottom, respectively).

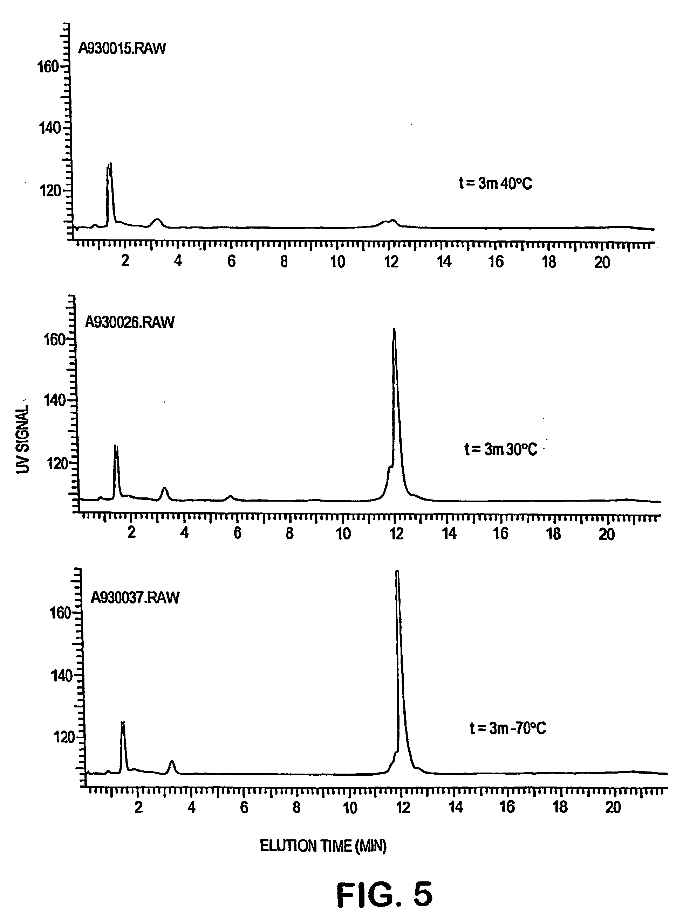

FIG. 5 shows chromatograms of reverse phase high performance liquid chromatography for the effects of polyphosphate on ala-TFPI stability. The aqueous formulations contained 5 mg/ml ala-TFPI, 10 mM L-histidine at pH 7 and 2.5 mg/ml polyphosphate. The aqueous formulations were stored for three months at 40°C, 30°C or -70°C (from top to bottom, respectively).

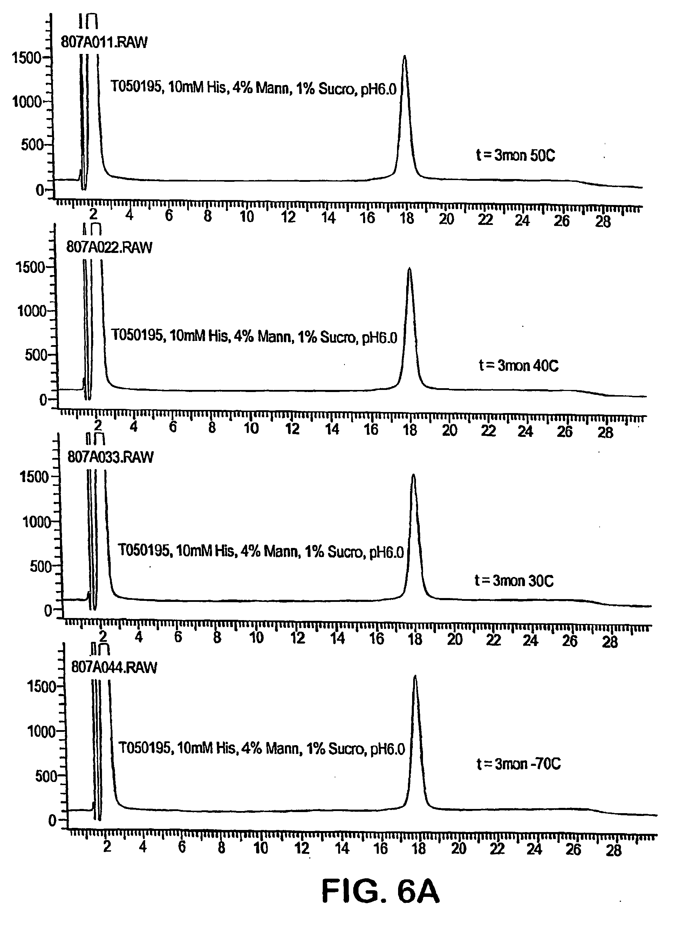

FIG. 6A shows cation exchange high performance liquid chromatography chromatograms of ala-TFPI samples prepared as lyophilized compositions. These lyophilized compositions contained 0.5 mg/ml ala-TFPI, 10 mM L-histidine at pH 6, 4% (w/v) mannitol and 1% (w/v) sucrose. Lyophilized compositions were stored for three months at 50°C, 40°C, 30°C or-70°C (from top to bottom, respectively).

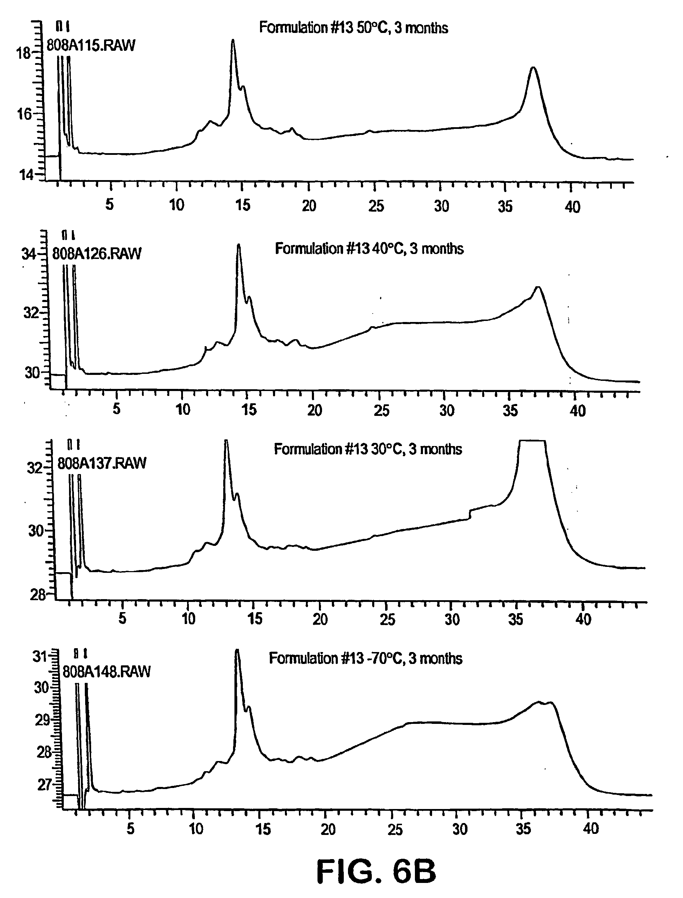

FIG. 6B shows reverse phase high performance liquid chromatography chromatograms of ala-TFPI samples prepared as lyophilized compositions. These lyophilized compositions contained 0.5 mg/ml ala-TFPI, 10 mM L-histidine at pH 6, 4% (w/v) mannitol and 1% (w/v) sucrose. Lyophilized compositions were stored for three months at 50°C, 40°C, 30°C or-70°C (from top to bottom, respectively).

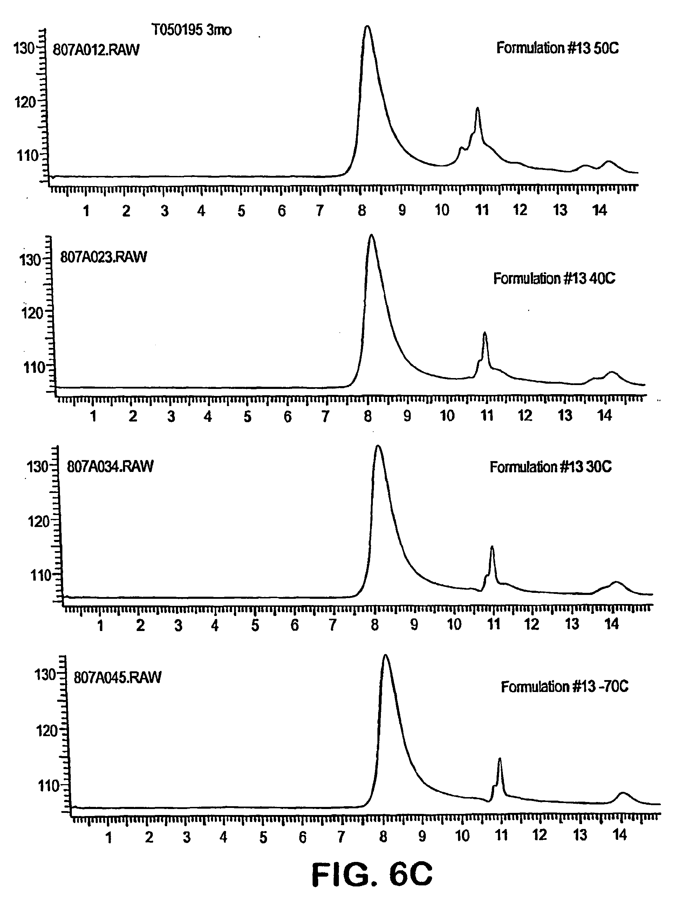

FIG. 6C shows size exclusion high performance liquid chromatography chromatograms of ala-TFPI samples prepared as lyophilized compositions. These lyophilized compositions contained 0.5 mg/ml ala-TFPI, 10 mM L-histidine at pH 6, 4% (w/v) mannitol and 1% (w/v) sucrose. Lyophilized compositions were stored for three months at 50°C, 40°C, 30°C or-70°C (from top to bottom, respectively).

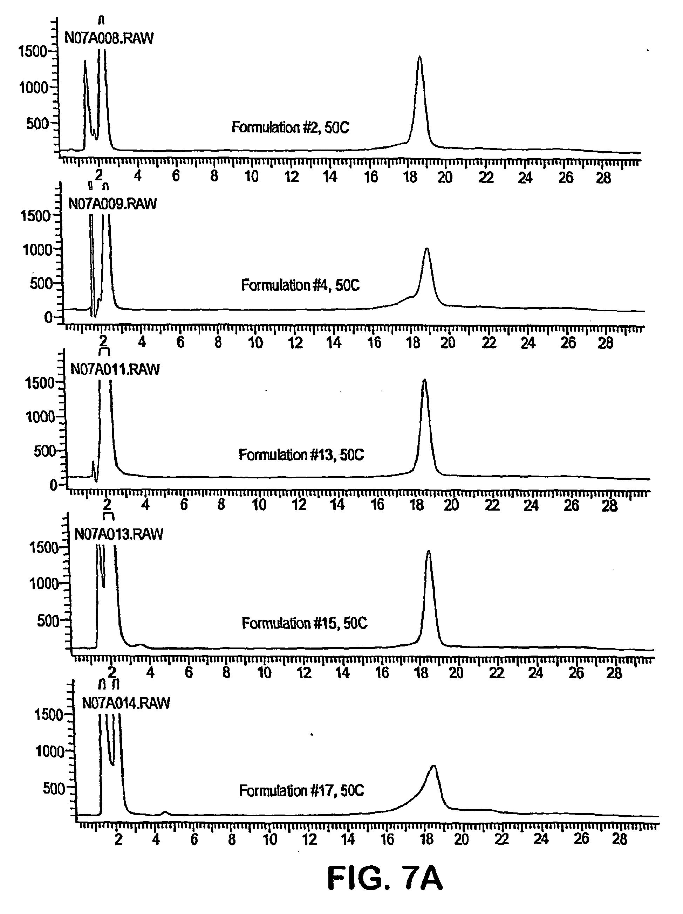

FIG. 7A shows chromatograms of cation exchange high performance liquid chromatography of ala-TFPI for testing stability of samples prepared as various lyophilized compositions (formulations 2, 4, 13, 15 and 17). See Table 4 for formulation compositions. The lyophilized compositions were stored for 6 months at 50°C.

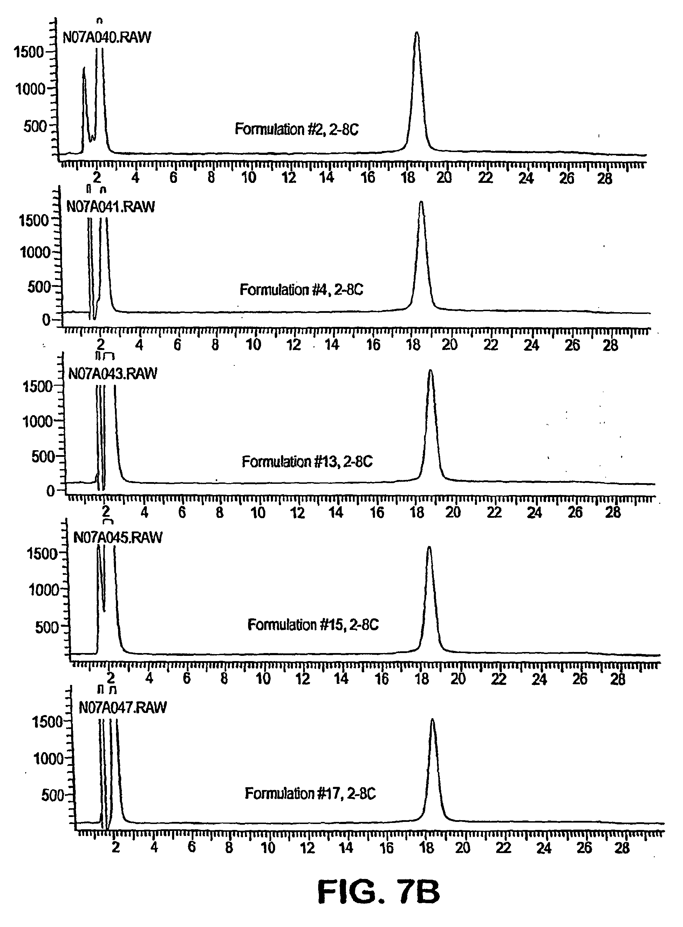

FIG. 7B shows chromatograms of cation exchange high performance liquid chromatography of ala-TFPI for testing stability of samples prepared as various lyophilized compositions (formulations 2, 4, 13, 15 and 17). See Table 4 for formulation compositions. The lyophilized compositions were stored for 6 months at 2-8°C.

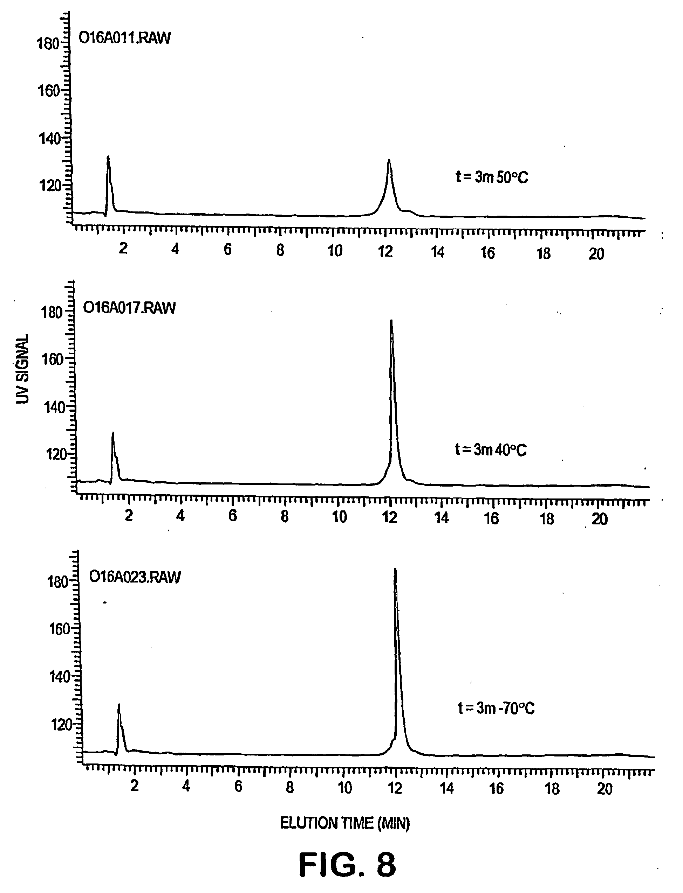

FIG. 8 shows chromatograms of reverse phase high performance liquid chromatography of ala-TFPI for testing stability of samples prepared as lyophilized compositions. The lyophilized compositions contained 5 mg/ml ala-TFPI, 10 mM L-histidine at pH 7, 4% (w/v) mannitol, 1% (w/v) sucrose and 2.5 mg/ml polyphosphate. Lyophilized compositions were stored for three months at 50°C, 40°C or -70°C (from top to bottom, respectively).

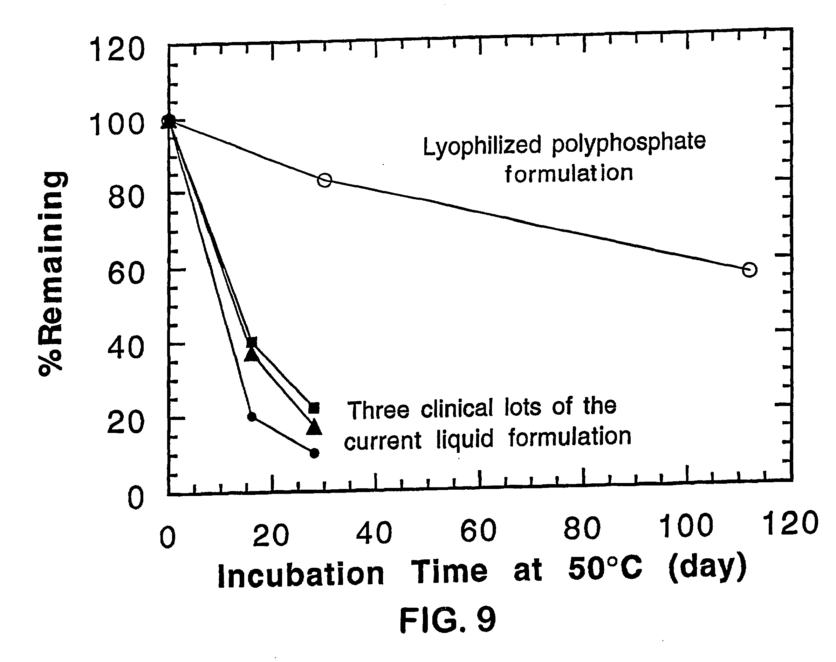

FIG. 9 shows a stability comparison of ala-TFPI lyophilized formulations compared with high concentration ala-TFPI aqueous formulations. The graph shows the percentage of soluble ala-TFPI remaining after storage at 50°C.

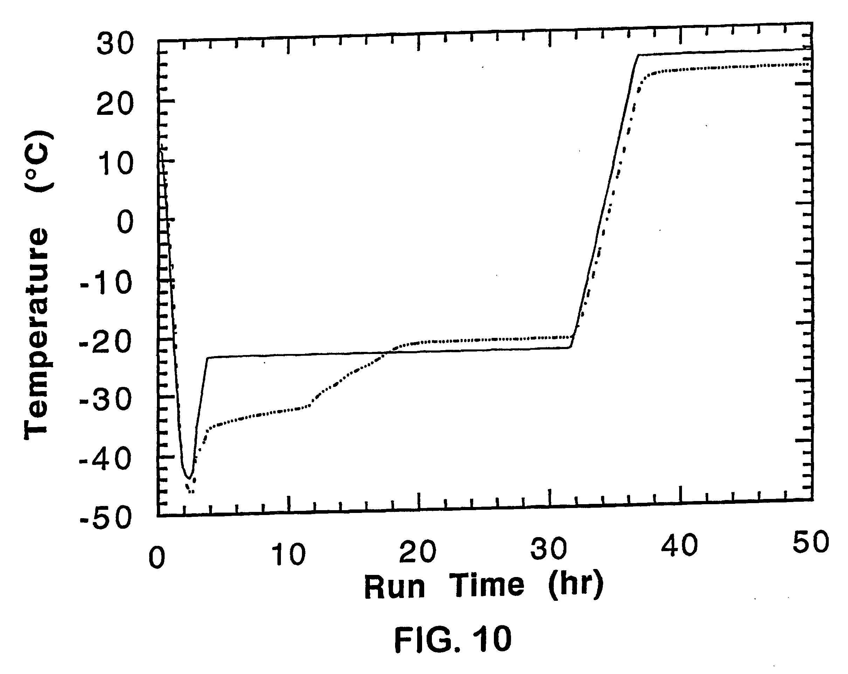

FIG. 10 shows the temperature record for freeze-drying of compositions containing ala-TFPI and various polyphosphate formulations. The continuous line indicates the shelf temperature during the course of the freeze-drying. The dotted line indicates the average temperature of the formulation as measured by four temperature probes inserted into the ala-TFPI/polyphosphate formulation s in 5 cc vials.

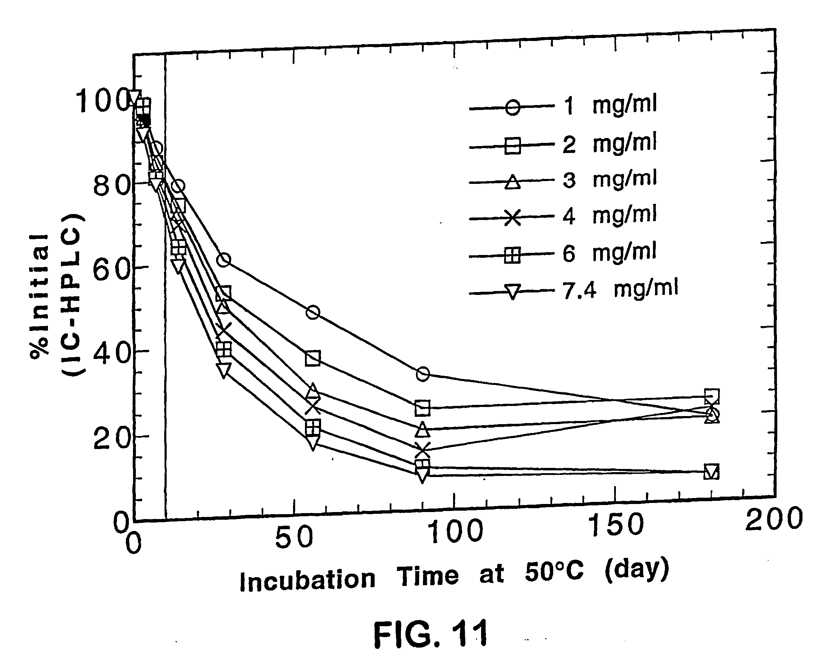

FIG. 11 shows the percent soluble ala-TFPI remaining following incubation at 50°C for up to six months. The aqueous formulations contained various amounts of ala-TFPI in 20 mM sodium citrate at pH 5.5 and 300 mM arginine. Following incubation at 50°C for the times indicated, soluble ala-TFPI was assayed by cation exchange HPLC to determine the amount of soluble ala-TFPI remaining in solution.

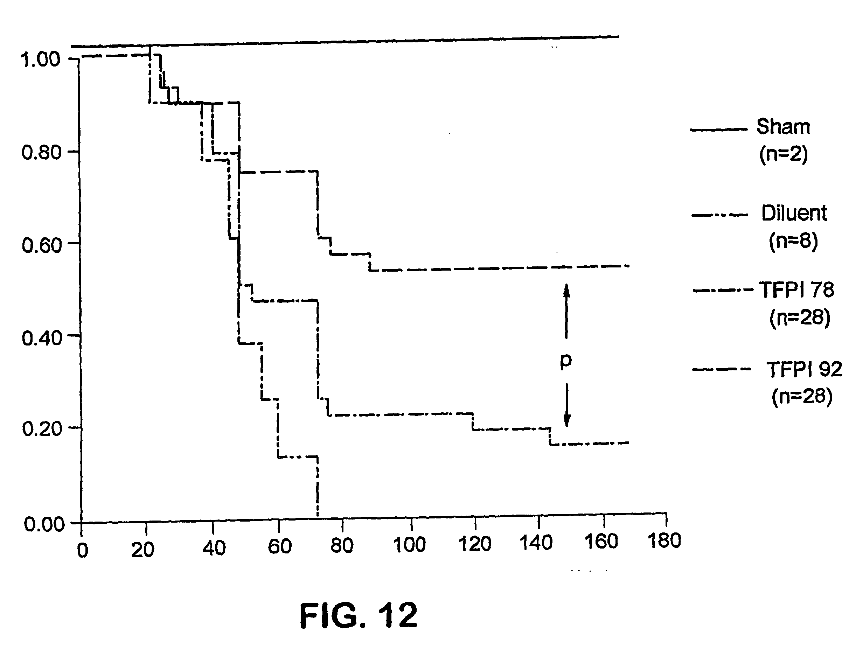

FIG. 12. Kaplan-Meier survival plots. X-axis, survival; Y-axis, time (hours).

### DETAILED DESCRIPTION OF THE INVENTION

High pH buffers favor aggregation of TFPI or TFPI variants in aqueous compositions. Aggregation of a TFPI or TFPI variant causes denaturation and inactivation of the molecule. Low pH buffers cause acid catalyzed hydrolysis of TFPI or TFPI variants in aqueous compositions. Thus, the optimal pH range for stable aqueous compositions of TFPI or TFPI variants is from about pH 4 to about pH 8. A TFPI or TFPI variant composition having a pH from about 4 to about 8 and containing a glass forming agent can be lyophilized to yield a highly stable composition.

Lyophilization ("freeze drying") is the removal of water from a formulation. Lyophilization can be carried out by any method known in the art, e.g., by use of a Hull Freeze Dryer, described below. Removal of water prevents water-dependent degradation reactions from occurring. Such reactions include, e.g., peptide bond hydrolysis and deamidation.

Lyophilized compositions of the invention can be made by lyophilizing an aqueous formulation comprising about 10 mg/ml or less of TFPI or TFPI variant (*i.e.,* 10, 7.5, 5, 2.5, 1, 0.5, or 0.2 mg/ml or less) and a glass forming agent. The aqueous formulation before lyophilization preferably has a pH from about 4 to about 8 *(i.e.,* 4, 4.5, 5, 5.5, 6, 6.5, 7, 7.5, or 8), more preferably a pH from about 5 to about 8, even more preferably a pH from about 5 to about 7, still more preferably a pH from about 5.5 to about 6.5, and even more preferably a pH of about 6. Optionally, the aqueous formulation can comprise a buffer and/or one or more crystal forming agents.

Other TFPI and TFPI compositions can be prepared by lyophilizing an aqueous formulation comprising the TFPI or TFPI variant and (a) a citrate buffer, (b) a mixture of sulfate and a phosphate buffer, or (c) sulfate and a buffer having a pH from about 4 to about 8. Components of the lyophilized compositions are described separately, below.

A preferred lyophilized product contains 20 mg/ml TFPI or TFPI variant, 10 mg/ml polyphosphate, 10 mM L-histidine at pH 7, 4% mannitol, and 1% sucrose.

### Aggregation stability of lyophilized compositions

Lyophilized compositions of the invention have about 45% or greater aggregation stability. "Percent aggregation stability" refers to the proportion of a TFPI or TFPI variant sample that is soluble as measured in a 40°C accelerated stability assay. In a 40°C accelerated stability assay, a TFPI or TFPI variant sample is incubated for three months at 40°C. Following incubation, the lyophilized TFPI or TFPI variant sample is reconstituted with sterile water, filtered through a 0.2 µm filter, and subjected to a cation exchange high performance liquid chromatography (CEX-HPLC) assay to determine the amount of soluble TFPI or TFPI variant remaining in solution. A CEX-HPLC assay is described below. Thus, for example, a TFPI or TFPI variant composition that has 60% aggregation stability is a composition in which 60% of the TFPI or TFPI variant is soluble as measured in the 40°C accelerated stability assay. A TFPI or TFPI variant composition that has 80% aggregation stability is a composition in which 80% of the TFPI or TFPI variant is soluble as measured in the 40°C accelerated stability assay. The percent aggregation stability of TFPI or TFPI variant compositions of the invention preferably is about 45, 50, 60, 70, or 75% or greater, more preferably about 80, 82, 84, 85, 90, 92, 94, 95, 96, 97, 98, or 99% or greater as measured in the 40°C accelerated stability assay and can range, for example, from about 45% or greater to about 90% or greater, about 45% or greater to about 96% or greater, 50% or greater to about 99% or greater, about 50% or greater to about 70% or greater, about 60% or greater to about 80% or greater, about 70% or greater to about 95% or greater, or about 85% or greater to about 96% or greater as measured in the 40°C accelerated stability assay. Preferably, the TFPI or TFPI variant in aqueous compositions of the invention is biologically active, as determined, for example, by a prothrombin time assay, as described below.

### Storage temperature

Storage temperatures for compositions of the invention can range from about -70°C to about 25°C (e.g., about -70, -60, -50, -40, -30, -20, -10, 0, 5, 10, 15, 20, or 25°C). Preferably, lyophilized compositions of the invention are stored at about 25°C.

### TFPI and TFPI variants

TFPI is a polypeptide having the amino acid sequence shown in SEQ ID NO:1. Preferably, TFPI is a recombinant human protein generated in a microbial host. TFPI is further characterized and described with respect to its biological activity in WO 01/24814.

TFPI variants include analogs and derivatives of TFPI, as well as fragments of TFPI, TFPI analogs, and TFPI derivatives. TFPI variants can be obtained from human or other mammalian sources, synthesized, or obtained by recombinant techniques. Analogs are TFPI molecules with one or more amino acid substitutions, insertions, deletions, and/or additions. Conservative substitutions, in which an amino acid is exchanged for another having similar properties, are preferred. Examples of conservative substitutions include, but are not limited to, Gly⇔Ala, Val⇔Ile⇔Leu, Asp⇔Glu, Lys⇔Arg, Asn⇔Gln, and Phe⇔Trp⇔Tyr. They typically fall in the range of about 1 to 5 amino acids (*i.e.*, 1, 2, 3, 4, or 5 amino acids). Additional amino acids can be added at any position in the molecule, particularly at the amino- or carboxy terminus. For example, one TFPI analog, N-L-alanyl-TFPI ("ala-TFPI"), has an additional alanine residue at the amino terminal end. Amino acid additions may be 1, 2, 5, 10, 25, 100, or more additional amino acids. Fusion proteins are encompassed within the definition.

Fragments are portions of TFPI, TFPI analogs, or TFPI derivatives. Examples of fragments include Kunitz domains 1, 2, or 3, Kunitz domains 1 and 2 or 2 and 3, or deletions of the N-terminus, C-terminus or both. Substantial guidance for making variants is found in U.S. 5,106,833. Fragments of TFPI comprise at least 20 consecutive amino acids of SEQ ID NO:1. For example, a fragment can be 20, 25, 30, 50, 100, 150, 200, 250, or 275 consecutive amino acids in length. TFPI fragments not possessing biological activity are described in U.S. 5,106,833. Use of such fragments in the present invention is also contemplated.

Derivatives are defined as TFPI, TFPI analogs, or TFPI fragments having additional moieties. Examples of such additions include glycosylation, phosphorylation, acetylation, or amidation.

Percent homology between a TFPI variant and SEQ ID NO:1 is determined using the Blast2 alignment program (Blosurn62, Expect 10, standard genetic codes, open gap 11, extension gap 1, gap x_dropoff 50, and low complexity filter off). TFPI variants will generally have at least about 70%, preferably at least about 80%, more preferably at least about 90% to 95% *(i.e.,* 90, 91, 92, 93, 94, or 95%) or more, and most preferably at least about 98% or 99% amino acid sequence identity to SEQ ID NO:1.

Amino acid sequence variants of TFPI can be prepared by making alterations in a DNA sequence encoding TFPI. Methods for making nucleotide sequence alterations are well known in the art. See, for example, Walker and Gaastra, eds. (1983) Techniques in Molecular Biology (MacMillan Publishing Company, New York), Kunkel (1985) Proc. Natl. Acad. Sci. USA 82:488-492, Kunkel et al. (1987) Methods Enzymol. 154:367-382, Sambrook et al. (1989) Molecular Cloning: A Laboratory Manual (Cold Spring Harbor, New York), U.S. 4,873,192,

TFPI variants preferably possess a substantial amount of biological activity, for example 10%, 30%, 50%, 60%, 80%, 90% or more of the biological activity of TFPI as measured in the PT assay described below. Obviously, any alterations made in the DNA encoding a TFPI variant must not place the sequence out of reading frame and preferably will not create complementary regions that could produce secondary mRNA structure. Guidance in determining which amino acid residues can be substituted, inserted, or deleted without abolishing biological or immunological activity of TFPI or TFPI variant can be found using computer programs well known in the art, such as DNASTAR software, or in Dayhoff et al. (1978) in Atlas of Protein Sequence and Structure (Natl. Biomed. Res. Found., Washington, D.C.). Stabilization of TFPI variants that are not biologically active also is contemplated.

TFPI or TFPI variants may be produced recombinantly as shown in U.S. 4,966,852. For example, a cDNA for the desired protein can be incorporated into a plasmid for expression in prokaryotes or eukaryotes. There are many references known to those skilled in the art that provide details on expression of proteins using microorganisms. See U.S. 4,847,201 and Maniatas et al., 1982, Molecular Cloning: A Laboratory Manual (Cold Spring Harbor, New York).

A variety of techniques are available for transforming microorganisms and using the transformed microorganism to express TFPI or TFPI variants. The following are merely examples of possible approaches. TFPI or TFPI variant DNA sequences can be connected to appropriate control sequences. TFPI or TFPI variant DNA sequences can be incorporated into a plasmid, such as pUC13 or pBR322, which are commercially available from companies such as Boehringer-Mannheim. Once the TFPI or TFPI variant DNA is inserted into a vector, it can be cloned into a suitable host. The DNA can be amplified by techniques such as those shown in U.S. 4,683,202 and U.S. 4,683,195. cDNA may be obtained by inducing cells, such as HepG2 or SKHep hepatoma cells, to make mRNA, then identifying and isolating the mRNA and reverse transcribing it to obtain cDNA. After the expression vector is transformed into a host such as *E. coli,* the bacteria may be cultured and the protein expressed. Bacteria are preferred prokaryotic microorganisms, and *E. coli* is especially preferred. A preferred microorganism useful in the present invention is *E. coli* K-12, strain MM294 deposited under the provisions of the Budapest Treaty on February 14, 1984 with the American Type Culture Collection, now located at 10801 University Blvd., Manassas, Virginia (Accession Number 39607).

TFPI or TFPI variants may be produced in bacteria or yeast and subsequently purified. Generally, procedures can be employed as shown in U.S. 5,212,091, U.S. 6,063,764, and U.S. 6,103,500 or WO 96/40784. TFPI or TFPI variants can be purified, solubilized, and refolded according to WO 96/40784 and Gustafson et al., Prot. Express. Pur. 5:233 (1994). For example, when prepared according Example 9 of WO 96/40784, preparations of ala-TFPI are obtained that contain from about 85% to 90% of the total protein by weight as biologically active ala-TFPI.

### Glass forming agents

A "glass forming agent" is a chemical agent with the ability to form glass below a critical temperature, the glass transition temperature (Tg). If a glass forming agent is lyophilized below its Tg, glass will form and will remain in the lyophilized composition. However, if the glass forming agent is lyophilized above Tg, then glass does not form. During the formation of glass, proteins can become embedded within the glass structure. Glass forming agents suitable for use with the present invention include, but are not limited to, charged polymers, monosaccharides, disaccharides, trisaccharides, and naturally occurring amino acids. Carbohydrate and amino acid glass forming agents are preferred. A combination of glass forming agents also is contemplated within a single formulation.

A "charged polymer" is any compound composed of a backbone of repeating structural units linked in linear or non linear fashion, some of which contain positively or negatively charged chemical groups. The repeating structural units may be organic or inorganic. The repeating units may range from two to several million.

Suitable charged polymers for use as glass forming agents include, but are not limited to, polyphosphate, heparins, dextran sulfates, agaropectins, alginic acids, carboxymethyl celluloses, polyinorganics, polyaminoacids, polyaspartates, polyglutamates, polyhistidines, polyorganics, DEAE dextrans, polyorganic amines, polyethyleneimines, polyethyleneimine celluloses, polyamines, polylysines, and polyarginines.

A "polyphosphate" is a polymer consisting of repeating units of orthophosphate linked in a phospho anhydride linkage. The number of repeating units can range from two (pyrophosphate) to several thousand. Polyphosphate is frequently referred to as sodium hexametaphosphate (SHMP). Other common names include Grahams salt, calgon, phosphate glass, sodium tetrametaphosphate, and glass H.

Monosaccharides used as glass forming agents include, but are not limited to, glycolaldehyde, glyceraldehyde, erythrose, threose, ribose, lyxose, xylose, arabinose, allose, talose, gulose, mannose, glucose, idose, galactose, altrose, dihydroxyacetone, erythrose, ribulose, xyloketose, psicose, tagatose, sorbose, and fructose. Sulfated monosaccharides may also be used.

Two monosaccharides linked together form a disaccharide. The two monosaccharides used to form a disaccharide can be the same or different. Examples of disaccharides which can be used as glass forming agents include, sucrose, trehalose, lactose, maltose, isomaltose, gentiobiose, laminaribiose, and cellobiose. Sulfated disaccharides may also be used.

Three monosaccharides linked together form a trisaccharide. The monosaccharides used to form a trisaccharide can be the same or different. Examples of trisaccharides suitable for use as glass forming agents include, raffinose and melezitose. Sulfated trisaccharides may also be used.

The naturally occurring amino acids suitable for use as glass forming agents in the present invention include, but are not limited to, glycine, alanine, valine, leucine, isoleucine, methionine, tyrosine, tryptophan, phenylalanine, lysine, serine, threonine, aspartic acid, glutamic acid, asparagine, glutamine, proline, cysteine, histidine, arginine, and any combination thereof. Amino acids may be either L- or D-stereo isomers. L-amino acids are preferred.

Glass forming agents are present in an amount from about 50 mM to about 600 mM *(i.e.,* about 50, 100, 150, 200, 250, 300, 350, 400, 450, 500, 550, or 600 mM) in an aqueous formulation for lyophilization. Preferably, glass forming agents are present in an amount from about 100 mM to about 500 mM, more preferably, from about 200 mM to about 400 mM, even more preferably from about 100 mM to about 300 mM, and most preferably about 300 mM.

Polymers, such as polyphosphate, generally are not well defined in terms of molecular weight. Some polymers are long while other are short so the molecular weight varies between the polymers. If a glass forming agent is not a well defined molecule, for example, a polymer such as polyphosphate, the amount present in the aqueous formulation is expressed as a ratio of the weight of TFPI or TFPI variant to the weight of charged polymer. Preferably, the TFPI or TFPI variant to charged polymer ratio is about 8:1 or less, more preferably, about 6:1, and most preferably about 2:1.

### Buffers

The pH of TFPI or TFPI variant compositions affects the solubility of the protein and hence its stability. See Chen et al. (1999) J. Pharm. Sciences 88(9):881-888. A preferred range of pH for the composition of the present invention is from about 4 to about 8 (*i*.*e*., about pH 4, 4.5, 5, 5.5, 6, 6.5, 7, 7.5, or 8), more preferably from about 5 to about 6.5. Because pH is a significant factor in TFPI solubility, use of a buffer to maintain the proper pH can additionally improve the stability of the formulations. Thus, aqueous compositions of the present invention optionally can further comprise a buffer to maintain solution pH.

Typical buffers suitable for use with the present invention include, but are not limited to, acetate, phosphate, succinate, glutamate, L-glutamate, imidazole, citrate, histidine, L-histidine, glycine, arginine, L-arginine, and a combination thereof. Preferred buffers are phosphate, L-glutamate, citrate, histidine, L-arginine and L-histidine. Most preferred buffers are L-arginine and L-histidine. Buffers are provided in the aqueous formulation in an amount from about 0 mM to about 600 mM (*i.e.*, about 0, 5, 10, 20, 50, 100, 150, 200, 250, 300, 350, 400, 450, 500, 550, or 600 mM). Preferably, the concentration of buffer is about 10 mM to about 100 mM and more preferably from about 20 mM to about 50 mM.

Some glass forming agents can also be buffers. An example of such a glass forming agent is arginine. Such glass forming agents buffer the pH of the aqueous formulation and, when the formulation is lyophilized, they form glass and help stabilize the TFPI or TFPI variant. Glass forming agents that also are buffers are typically present in an amount from about 50 mM to about 600 mM (*i.e.,* about 50, 100, 150, 200, 250, 300, 350, 400, 450, 500, 550, or 600 mM) in the aqueous formulation for lyophilization. Preferably, the concentration of glass forming agent buffer is about 100 mM to about 500 mM, more preferably, from about 200 mM to about 400 mM, even more preferably from about 100 mM to about 300 mM, and most preferably about 300 mM.

### Crystal forming agents

Optionally, the aqueous formulation can also comprise one or more crystal forming agents. A "crystal forming agent" is a chemical agent with the ability to form a crystal lattice network. Typically, crystal forming agents are added to an aqueous formulation to be lyophilized to provide a rigid structure. The material remaining post-lyophilization is termed a "cake." Without a crystal forming agent present, the cake generally will not be rigid and will usually collapse or shrink, which can be detrimental to cake morphology. The rigid support of a crystal lattice will prevent the collapse of the cake. Cake morphology is desirable from a product appearance aspect. The stability and bioactivity of lyophilized TFPI or TFPI variant generally is unaffected by cake morphology.

Examples of crystal forming agents suitable for use with the present invention include, but are not limited to, mannitol, alanine, glycine, sodium chloride, and any combination thereof. The crystal forming agents are present in the aqueous formulation for lyophilization in an amount from about 0.5% to about 16% (weight/volume) (*i.e.,* about 0.5, 1, 2, 3, 4, 5, 6, 7, 8, 9, 10, 11, 12, 13, 14, 15, or 16%).

### Preparation of formulations for lyophilization

Bulk preparation of TFPI or TFPI variant may involve using a chaotrope, such as urea, to ensure that the TFPI or TFPI variant remains soluble. It may be desirable or required to remove the chaotrope prior to lyophilization. Thus, the invention also provides a process to aid in preparing a composition of TFPI or TFPI variant for lyophilization.

The first step in the process is to remove a first formulation low molecular weight solute, usually the chaotrope, and replace it with a second formulation low molecular weight solute such as sodium citrate, sodium phosphate, arginine, histidine, mannitol, and sucrose. To prevent aggregation of the TFPI or TFPI variant as the first formulation low molecular weight solute is removed, the pH is lowered to a pH value, which permits removal of the chaotrope without aggregation or precipitation of the TFPI or TFPI variant protein. Usually, the pH is reduced to about 4. The chaotrope can be removed by any method known in the art. Examples of such methods include, but are not limited to diafiltration, dialysis, or size exclusion chromatography. Diafiltration is preferred. It may be desirable to exchange solutes in a bulk preparation and lyophilize the bulk TFPI or TFPI variant prior to storage.

Alternatively, the concentration of the chaotrope can be reduced by diluting the TFPI or TFPI variant preparation with a formulation that does not contain the chaotrope. This step results in the formation of a formulation which is preferably, but not necessarily, essentially chaotrope-free.

If desired, some solutes in the resultant formulation can be exchanged for other solutes (e.g., histidine, imidazole, mannitol, glycine, or sucrose). The pH is usually increased during this step to a value at which TFPI is stable in aqueous formulations. Preferably, the pH is increased to about 6. Optionally, the formulation can subsequently be lyophilized by techniques known in the art. The third formulation is preferably a pharmaceutically acceptable formulation. The term "pharmaceutically acceptable" means there are no significant adverse biological effects when the formulation is administered to a patient. The term "patient" encompasses both human and veterinary patients.

Lyophilized compositions of the present invention can be reconstituted to provide an aqueous formulation of TFPI or TFPI variant. The lyophilized TFPI can be reconstituted with a suitable medium, for example, saline solution or water.

The following examples are offered by way of illustration.

### EXAMPLE 1

### General Methods

### Bulk Preparation of TFPI or TFPI Variant

ala-TFPI bulk was prepared into different formulation buffers by dialysis. Dialysis was carried out at 4°C using Spec/Por7 dialysis tubing with a molecular weight cutoff of 3,500 daltons. dialysis buffer at 50- to 100-fold excess was renewed every three hours for a total of three times. After dialysis, soluble ala-TFPI was separated from aggregated TFPI by filtration through a 0.2 µm filter unit. The concentration of soluble ala-TFPI was measured by UV absorbance and was adjusted to desired values according to formulation needs. In a sterile laminar flow hood, 1 ml of the formulated ala-TFPI solution was transferred to 3 cc type I glass vials. These vials were then stoppered with 13 mm Daikyo D713 fluoro-resin laminated rubber stoppers, cramped with aluminum seals, and placed into stability chambers. For lyophilization formulation, 3 cc glass vials were filled with 1 ml of formulated ala-TFPI solutions and were stoppered half way with 13 mm West 890 Gray lyophilization stoppers. These vials were loaded into a lyophilizer for freeze-drying. *HPLC*

High performance liquid chromatography (HPLC): All HPLC methods were performed on a Waters 626 LC system with a 717 heater/cooler autosampler. UV absorbance was monitored by a Waters 486 absorbance detector, and fluorescence was recorded by a Hitachi F-1050 or F-1080 fluorescence spectrophotometer. Data acquisition and processing were performed on a Perkin-Elmer Turbochrom system.

Cation Exchange HPLC (CEX-HPLC): The CEX-HPLC method used a Pharmacia Mono-S HR 5/5-glass column. The column was equilibrated in 80% buffer A (20 mM sodium acetate trihydrate:acetonitrile solution (70:30 v/v) at pH 5.4) and 20% buffer B (20 mM sodium acetate trihydrate-1.0 M ammonium chloride-acetonitrile solution (70:30 v/v) at pH 5.4). After a sample was injected, a gradient was applied to elute the TFPI at a flow rate of 0.7 ml/min from 20% buffer B to 85% buffer B in 21 minutes. Protein peaks were detected by absorbance at 280 nm or fluorescence using an excitation 280 nm and emission 320 nm.

Size Exclusion HPLC (SEC-HPLC): The SEC-HPLC method used a Phenomenex BIOSEP-SECS2000 column (300 x 7.8 mm). The ala-TFPI was eluted from the column using an isocratic elution profile. ala-TFPI was eluted with 10 mM sodium phosphate at pH 6.5 and 0.5 M NaCl. Protein peaks were detected by absorbance at 278 nm, and protein molecular weights were determined by fluorescence using excitation at 467 nm and emission at 467 nm *(*Dollinger et al., J. Chromatogr. 592:215-228, 1992).

Reverse Phase HPLC (RP-HPLC): The reverse phase HPLC method used two Rainin Dynamax C8 columns (5 cm x 4.6 mm, 5 µm, 30nm (300 Å) in series. The column was preequilibrated in 86% buffer A (30% (v/v) acetonitrile:0.45% (v/v) trifluoroacetic acid) and 14% buffer B (60% (v/v) acetonitrile:0.45% (v/v) trifluoroacetic acid). Injected protein was eluted by first increasing buffer B to 21 % in 13 min and then to 100% buffer B in 30 min at a flow rate of 1 ml/min. Protein elution was detected by measuring the absorbance at 280 nm.

### Prothrombin Time (PT) Assay

The PT assay was performed on a Coag-A-Mate RA4 instrument (Organon Teknika). ala-TFPI samples were first diluted to 150 µg/ml with buffer (2 M urea, 20 mM sodium phosphate, 250 mM NaCl, pH 7.2), then to 30 µg/ml with TBSA buffer (50 mM Tris, 100 mM NaCl, 1 mg/ml bovine serum albumin, pH 7.5) and finally to 12 to 15 µg/ml by TBSA buffer. For assay, 10 µl of diluted sample was first mixed with 90 µl of pooled Verify I (Organon Teknika, Cat. No. 59566), loaded on a test tray (Organon Teknika, Cat. No. 35014), and placed into the Coag-A-Mate. Then 200 µl of Simplastin Excel (Organon Teknika, Cat. No. 52001) was added to initiate the clotting process. The clotting time was converted to the input ala-TFPI concentration by comparing with a standard plot of the log of the clotting time in seconds versus the log of the ala-TFPI concentration in the standards.

### Addition of PEI

PEI was added to ala-TFPI/polyphosphate samples to eliminate interference of polyphosphate on the RP-HPLC analysis and the PT assay. Without PEI, ala-TFPI showed abnormal HPLC elution profile and reduced *in vitro* specific activity (0.6-0.9) in the presence of polyphosphate. Addition of 0.8% (w/v) PEI restored the normal elution profile and the normal *in vitro* specific activity. ala-TFPI precipitation was observed with PEI was added at concentrations of 0.2 and 0.4% (w/v). PEI at concentrations of 0.6 to 3.2% (w/v) caused no ala-TFPI precipitation and had no effect on the clotting time in the PT assay when used alone.

### Diafiltration

Diafiltration: TFPI or TFPI variant bulk which contained about 10 mg/ml TFPI or TFPI variant, 2 M urea, 20 mM sodium phosphate at pH 7 and 150 mM NaCl was first mixed with 1.25 to 5 mg/ml polyphosphate (TFPI to polyphosphate weight ratios varied from about 8:1 to about 2:1). Urea was added to 6 M. The diafiltration membrane was a Millipore Pellicon 2 mini 10 K PLGC regenerated cellulose membrane with 0.1 m² surface area. Inlet pressure, outlet pressure, and flow rate of the peristaltic pump were set according to manufacturer recommendations.

Diafiltration Efficiency: If the solution volume in the diafiltration vessel is kept constant during the entire diafiltration process (*i.e.*, the volume of feed-in diafiltration buffer is equal to the volume of permeate-out solution), an exchange equation can be used to calculate the diafiltration efficiency at each volume of buffer exchange:
Cn/Co = exp(-α,Vn/Vo)

Here, Cn and Co are the concentrations of a solute in the diafiltration vessel (but not present in the feed-in buffer) at n and zero volumes of buffer exchange, respectively. Vo is the volume of the starting TFPI or TFPI variant formulation in the diafiltration vessel, and Vn is the volume of buffer fed into or diafiltration solution permeated out. Thus, n = Vn/Vo. The diafiltration cartridge used a membrane with a molecular weight cutoff of 10 kilodaltons, which was assumed to be completely permeable to small solutes such as urea, NaCl and phosphate. Therefore, the permeation factor a equals 1 for small solutes well below the molecular weight cutoff. This equation allows the calculation of the diafiltration efficiency at each volume of buffer exchange. For example, 98.2% and 99.8% starting buffer components will be "permeated out" after 4 and 6 volumes of buffer exchange, respectively.

### Dialysis

In some experiments, TFPI or TFPI variant bulk was prepared in different formulation buffers by dialysis. Dialysis was carried out at 4°C using Spectra/Por7^{®} dialysis tubing (Spectrum^{®} Laboratories) with a molecular weight cutoff of 3,500 daltons. Dialysis buffer at 50- to 100-fold excess was replaced every three hours for a total of three times. After dialysis, soluble TFPI or TFPI variant was separated from aggregated TFPI or TFPI variant by filtration through a 0.2 µm filter unit. The concentration of soluble TFPI or TFPI variant was measured by UV absorbance and was adjusted to desired values according to formulation needs.

### Lyophilization (Freeze-drying)

Vials containing formulated TFPI or TFPI variant were placed onto metal trays. These trays were loaded into a Hull Freeze Dryer (Model 8FS 12C) with a shelf temperature preequilibrated at 10°C. The freeze-drying cycle was composed of three steps: freezing, primary drying, and secondary drying. The vials were first cooled down to -50°C to freeze the products. The primary drying was subsequently carried out at -25°C for 30 hours at a vacuum of 135 m bar (100 mtorr). The secondary drying was then conducted at 20°C for 12 hours, also at a vacuum of 135 mbar (100 mtorr). After the freeze-drying was completed, the shelf temperature was lowered to 4°C and the vacuum was released by bleeding nitrogen into the lyophilizer chamber. At a 827 mbar (12 psi) nitrogen pressure, vials were stoppered. These vials were withdrawn from the lyophilizer and crimped with 13-mm flip-off aluminum seals.

### EXAMPLE 1A (reference example)

### Diafiltration of TFPI/polyphosphate

An ala-TFPI/polyphospbate solution was prepared by added polyphosphate into an ala-TFPI bulk (containing 2 M urea, 20 mM sodium phosphate, and 150 mM NaCl) before diafiltration. This mixture was then diafiltered against water or buffers. Urea at 6M was used during diafiltration to keep the protein soluble during the buffer exchange process.

The diafiltered ala-TFPI/polyphosphate solution showed good stability. No precipitation was observed during subsequence storage at ambient temperature. In contrast, ala-TFPI/polyphosphate solutions prepared directly from the bulk buffer (containing only 2 M urea) showed instability after subsequent storage; precipitate was formed after one day of storage at ambient temperature. Thus, 6 M urea helps ala-TFPI solubilization during buffer transfer from the bulk buffer to the water/polyphosphate buffer.

### EXAMPLE 1B (reference example)

### Freezing-Induced ala-TFPI Precipitation

The diafiltered ala-TFPI/polyphosphate solution showed instability upon either freeze-thawing or incubation at ambient temperature. Visible precipitate was found in some ala-TFPI/polyphosphate solutions after being freeze-thawed from -70°C or during storage at ambient temperature, as shown in Table A.

**Table A.**

| Formulation condition | Visual observation of ala-TFPI precipitation (and pH) | |
|---|---|---|
| | RT storage | Freeze-thawed from -70°C |
| Preparation 1 (7.4 mg/ml ala-TFPI) | | |
| in water | clear (pH 6.5-7) | clear (pH 6, frozen) |
| 10 mM L-histidine | clear (pH 6.5-7) | clear (pH 7, frozen) |
| 10 mM sodium phosphate | cloudy (pH 6.5-7) | clear (pH 5-5.5, frozen) |
| 10 mM sodium citrate | | cloudy (pH 5.5-6, frozen) |
| Preparation 2 (23 mg/ml ala-TFPI) | | |
| in water | cloudy (pH 7) | clear (pH 5.5-6, frozen) |
| 10 mM sodium phosphate | cloudy (pH 7) | hazy (pH 6.5-7, frozen) |
| 10 mM sodium citrate | very cloudy (pH 7) | cloudy (pH 5-5.5, frozen) |
| Preparation 3 (22 mg/ml ala-TFPI) | | |
| in water | clear (pH 6.5-7) | hazy (pH 6-6.5, frozen) |
| 10 mM L-histidine | clear (pH 6.5-7) | hazy (pH 6.5-7, frozen) |
| 10 mM sodium phosphate | hazy (pH 6.5) | cloudy (pH 5.5-6, frozen) |
| 10 mM sodium citrate | cloudy (pH 6.5) | cloudy (pH 6, frozen) |

These results show that ala-TFPI stability is sensitive to the storage conditions and buffers in the formulation. Citrate and phosphate buffers often caused ala-TFPI precipitation both at ambient temperature storage and at frozen storage. To assess if pH had changed at -70°C, pH at the frozen state was measured by the dye color change using the Universal pH solution purchased from Fisher Scientific.

The ala-TFPI/polyphosphate solution was found to be extremely sensitive to changes in both the ionic strength and pH. Titrated by either acid or NaCl, one experiment showed that TFPI precipitated immediately in ala-TFPI/polyphosphate solutions either at pH below 5.8 or above 80 mM NaCl. Another experiment showed ala-TFPI eventually formed precipitate at ambient temperature after addition of NaCl as low as 5 mM NaCl. Addition of more NaCl resulted in a faster precipitation. The sensitivity of ala-TFPI/polyphosphate solutions to changes in pH and salt in the presence of polyphosphate may explain why ala-TFPI precipitated upon freeze-thawing. pH of buffers such as citrate and phosphate decreases upon freezing because of partial precipitation of different salt forms in the buffer system. Possibly, pH shift and/or concentration of salt upon freezing weakens interaction between ala-TFPI and polyphosphate and results in ala-TFPI precipitation. Similarly, addition of salt at ambient temperature could also weaken the electrostatic interaction between ala-TFPI and polyphosphate and result in ala-TFPI precipitation.

L-histidine is a useful buffer for stabilizing pH upon freezing. L-histidine also exists as either a simple neutral form or a protonated form in solutions. Therefore, it should have a minimal effect on the interaction between ala-TFPI and polyphosphate.

### EXAMPLE 2 (reference example)

### Stability of ala-TFPI Aqueous Compositions

To determine the long term stability of ala-TFPI aqueous formulations, aggregation of ala-TFPI was studied at elevated temperatures. Ala-TFPI samples formulated with 150 mM sodium chloride, 0.015% (w/v) polysorbate-80 and 10 mM sodium phosphate having a pH of 7 were stored at 40°C for various times. Turbidity in the samples developed with increasing storage times, indicating formation of visible precipitates. After filtration through a 0.2 µm filter, the soluble ala-TFPI was analyzed by cation exchange-HPLC (CEX-HPLC) as described above. FIG. 1 shows the chromatograms of CEX-HPLC for these samples. The chromatograms in FIG. 1 show that a single protein peak eluted at 18 minutes. This peak is ala-TFPI monomer.

Ala-TFPI concentration was calculated by integrating the area under the peaks in the chromatograms of FIG. 1. A decrease in the peak area was observed, indicating loss of soluble ala-TFPI in these samples. A single exponential fitting of the peak area versus storage time was used to calculate the loss of soluble ala-TFPI. The calculated rate constant was converted to the shelf life, t₉₀ (time taken for loss of 10% of the soluble ala-TFPI polypeptide), using the standard first order kinetic equation (Cantor and Schimmel, eds., Biophysical Chemistry, W.H. Freeman and Co., 1980). Similarly, the shelf-life was calculated using bioactivity data determined by the prothrombin time (PT) assay. The shelf-life for soluble ala-TFPI was in good agreement with the shelf-life for bioactivity (Table 1). Since loss of soluble ala-TFPI was in parallel to the decrease in bioactivity, aggregation was considered to be the most likely inactivation pathway.

**Table 1**

| Formulation | t₉₀ at 40°C (days) | |
|---|---|---|
| | CEX-HPLC | PT assay |
| 10 mM sodium acetate, 150 mM NaCl, pH 5.5 | 5.0 | 4.2 |
| 10 mM sodium citrate, 150 mM NaCl, pH 5.5 | 4.4 | 4.4 |
| 10 mM sodium acetate, 8% (w/v) sucrose, pH 5.5 | 5.3 | 4.6 |
| 10 mM sodium acetate, 4.5% (w/v) mannitol, pH 5.5 | 5.8 | 5.3 |
| 10 mM sodium succinate, 150 mM NaCl, pH 6.0 | 3.6 | 3.5 |
| 10 mM sodium citrate, 150 mM NaCl, pH 6.0 | 4.9 | 3.9 |
| 10 mM sodium phosphate, 150 mM NaCl, pH 6.0 | 4.2 | 3.3 |
| 10 mM sodium phosphate, 500 mM NaCl, pH 6.0 | 15.7 | 7.8 |
| 10 mM sodium citrate, 180 mM L-arginine, pH 6.0 | 12.7 | 6.5 |
| 10 mM sodium citrate, 150 mM NaCl, pH 6.0 | 4.1 | 3.5 |
| 10 mM sodium citrate, 50 mM L-arginine, pH 6.5 | 1.8 | 1.9 |
| 10 mM sodium phosphate, 150 mM NaCl, 0.015% (w/v) polysorbate-80, pH 7.2 | 1.0 | 1.5 |

To reduce aggregate formation and hence increase long-term stability of the ala-TFPI aqueous formulations, a pH stability study was conducted. Ala-TFPI was prepared in 150 mM sodium chloride, 0.015% (w/v) polysorbate-80 and 10 mM sodium phosphate, and the pH of the formulation was adjusted to different values between 4 and 9 by addition of HCl or NaOH. After storage at 40°C, loss of soluble ala-TFPI in these samples was determined by CEX-HPLC. FIG. 2 shows a plot of the first order rate constant for loss of soluble ala-TFPI as a function of pH. The aggregation rate was slower below pH 6 and became much faster at basic pH. Thus, ala-TFPI aggregation was base catalyzed.

Although the aggregation reaction was minimized below pH 6, a new degradation reaction was observed at acidic pH conditions. Two smaller species of ala-TFPI were detected by SDS-PAGE (data not shown). A late eluting species was also observed at pH 4 on CEX-HPLC chromatograms (FIG. 3). This species seemed to correspond to the degraded products detected by SDS-PAGE. FIG. 3 shows the monomeric ala-TFPI eluting at 18 minutes and a new species emerging with longer incubation of ala-TFPI in acidic conditions at 40°C. Thus, while the monomeric ala-TFPI species decreased with the incubation time, the late eluting peak presumably degraded ala-TFPI increased.

To determine if the degradation of ala-TFPI was catalyzed by acid hydrolysis, samples of ala-TFPI at different pH were subjected to CEX-HPLC analysis. The rate of cleavage became slower at higher pH conditions (FIG. 4). After being stored for 10 days at 40°C, cleavage of TFPI increased as pH decreased to pH 4. Therefore, the degradation of ala-TFPI is due to acid hydrolysis of peptide bonds within the polypeptide.

Based on the results of the previous two studies, further formulation screening focused around pH 6. Accelerated stability of 11 additional formulations, which are listed in Table 1 was evaluated. Samples were stored at 40°C and were analyzed by CEX-HPLC analysis for loss of solubility and PT assay for loss of bioactivity. The shelf-life (t₉₀), as determined by cation exchange HPLC, was found to range from one day to over two weeks and from about one day to about eight days as determined by the PT assay. The Arrehnius analysis was performed using these 40°C data, assuming a 10 kcal/mol activation energy (which is a reasonable estimation for an aggregation reaction). The results suggest that TFPI in these aqueous formulations is not stable for the longer time periods, e.g., 18-24 months.

### EXAMPLE3 (Reference example)

### Stabilizing Effect of Polyphosphate on Aqueous Formulations of Ala-TFPI

The stabilizing effect of polyphosphate on ala-TFPI formulations was evaluated. Polyphosphate binds to ala-TFPI and stabilizes it conformationally. Polyphosphate stabilization was studied at weight ratios of ala-TFPI to polyphosphate of 2:1, 6:1 and 8:1. Formulations were prepared at each ala-TFPI:polyphosphate ratio in 10 mM histidine at pH 7. In addition, to assess the effect of histidine on ala-TFPI stability, an 8:1 ala-TFPI to polyphosphate weight ratio was set up using water only. In yet another formulation, an 8:1 ala-TFPI to polyphosphate weight ratio was set up with 10 mM histidine having a pH of 6.5 to assess the effects of pH on ala-TFPI stability.

Because salt and other charged ions can interfere with ala-TFPI-polyphosphate binding, mono- and disaccharides such as mannitol, sucrose, and sorbitol were used in the TFPI formulations. Formulations containing mono- or disaccharides were prepared at an ala-TFPI to polyphosphate weight ratio 8:1 (see Table 2 for formulations). Finally, several high concentration ala-TFPI samples were set up to assess TFPI stability at such concentrations. The stability of these ala-TFPI formulations was examined at 30°C and 40°C. Vials were withdrawn at preselected time points and filtered through 0.22 µm filters to remove precipitate. The filtered ala-TFPI samples were subsequently analyzed by SDS-PAGE for degradation and band intensity, by reverse phase HPLC as described above for loss of solubility, and by the PT assay as described above for loss of bioactivity.

The SDS-PAGE results showed no cleavage of ala-TFPI but did show that band intensity was decreased for the formulations stored at 40°C for 3 months. Results of the RP-HPLC analysis are shown in FIG. 5. FIG. 5 shows chromatograms of an aqueous polyphosphate formulation incubated at 40°C or 30°C and frozen -70°C for three months. Ala-TFPI eluted around 12.2 minutes, and minor species eluted either ahead or behind the main species. Upon incubation at elevated temperatures (30°C or 40°C), visible precipitate developed in the samples. The visible precipitate could be filtered out through 0.22 µm filters and as such, the chromatograms showed decreased peak area without the emergence of new species. The results indicate that ala-TFPI was lost due to aggregation upon storage at 30°C or 40°C.

Aggregation during incubation at elevated temperatures was identified as the major degradation pathway for ala-TFPI at pH above 6. The concentration of soluble ala-TFPI was calculated by integrating the peak area on the RP-HPLC chromatograms (FIG. 5). To calculate percentage of remaining soluble ala-TFPI, these data were normalized to those for the same formulations stored at -70°C. Results are shown in Table 2. Formulation 1 contained water only and was more stable than the corresponding formulation in 10 mM histidine (formulation 2). At 30°C, a 15% difference in the remaining soluble protein was observed for samples with and without histidine. The 30°C data also show that the apparent decrease in stability due to histidine can be compensated for by addition of mannitol and/or sucrose (formulations 4, 5 and 7). Mannitol and sucrose are weak stabilizers for ala-TFPI in the presence of histidine. The interaction between ala-TFPI and histidine and ala-TFPI and mannitol or sucrose is expected to be additive *(*Chen et al., J. Pharm Sci 85:419-422, 1996).

**Table 2**

| Sample number | ala-TFPI to polyphosphate weight ratio | Formulation | Percent Soluble TFPI Remaining | |
|---|---|---|---|---|
| | | | 30°C | 40°C |
| 1 | 8:1 | water (pH 7) | 76.6 | 1.1 |
| 2 | 8:1 | 10 mM histidine, pH 7 | 61.9 | 0.9 |
| 3 | 8:1 | 10 mM histidine, pH 6.5 | 33.0 | 0.2 |
| 4 | 8:1 | 10 mM histidine, pH 7, 5% (w/v) mannitol | 75.9 | 0.8 |
| 5 | 8:1 | 10 mM histidine, pH 7, 9% (w/v) sucrose | 75.0 | 1.4 |
| 6 | 8:1 | 10 mM histidine, pH 7, 5% (w/v) sorbitol | 68.9 | 0.9 |
| 7 | 8:1 | 10 mM histidine, pH 7, 4% (w/v) mannitol, 1% (w/v) sucrose | 74.3 | 0.9 |
| 8 | 8:1 | 10 mM histidine, pH 7 | 77.8 | 2.7 |
| 9 | 2:1 | 10 mM histidine, pH 7 | 80.0 | 6.3 |
| 10 | no polyphosphate | high concentration ala-TFPI sample 1 (5 mg/ml) | 76.7 | 43.8 |
| 11 | no polyphosphate | high concentration ala-TFPI sample 2 (5 mg/ml) | 85.3 | 41.1 |

Stability of ala-TFPI-polyphosphate formulations strongly depends on pH. The 30°C data of Table 2 show that ala-TFPI stability was decreased by one-half when the pH was changed from 7 to 6.5 (formulations 2 and 3). In addition, ala-TFPI stability in polyphosphate formulations depends on the ratio of ala-TFPI to polyphosphate. The 40°C data of Table 2 show the percent of soluble ala-TFPI remaining to be 0.9, 2.7 and 6.3 when the ala-TFPI to polyphosphate ratio changes from 8:1 to 6:1 to 2:1 (formulations 2, 8, and 9), respectively. Thus, ala-TFPI is most stable at the ala-TFPI to polyphosphate weight ratio of 2:1.

The 30°C data show little difference in stability between polyphosphate formulations (formulations 1-9) and the high concentration ala-TFPI formulation (formulations 10 and 11). The 40°C data show that the high concentration TFPI formulations (formulations 10 and 11) are more stable than the TFPI-polyphosphate formulations. After 3 months storage, polyphosphate formulations (formulations 1-9) have less than 10% TFPI left, while formulations 10 and 11 have more than 40% TFPI remaining. Thus, an alternative method of achieving long term stability, *e.g.,* for 18-24 months, is to use high concentration TFPI formulations.

### EXAMPLE 4 (Reference example)

### Stability of Ala-TFPI in a Freeze-Thaw Cycle

To determine if the ala-TFPI formulations could be stored at -70°C, ala-TFPI-polyphosphate (6:1 w/w) formulations (Table 3) were subjected to a freeze-thaw cycle. Visible precipitate was found in some ala-TFPI-polyphosphate formulation s after being frozen at -70°C and thawed, as shown in Table 3. The results show that citrate and phosphate buffers can cause TFPI precipitation both at ambient temperature storage and at -70°C storage.

**Table 3**

| Formulation | Visual observation of ala-TFPI precipitation and pH value of the formulation | | | |
|---|---|---|---|---|
| | Room temperature | | Freeze-thaw cycle (from -70°C) | |
| | Clarity | pH | Clarity | pH of frozen formulation |
| Preparation 1 (7.4 mg/ml ala-TFPI) | | | | |
| in water | clear | 6.5-7 | clear | 6 |
| 10 mM L-histidine | clear | 6.5-7 | clear | 7 |
| 10 mM sodium phosphate | cloudy | 6.5-7 | clear | 5-5.5 |
| 10 mM sodium citrate | | | cloudy | 5.5-6 |

| Preparation 2 (23 mg/ml ala-TFPI) | | | | |
|---|---|---|---|---|
| in water | cloudy | 7 | clear | 5.5-6 |
| 10 mM L-histidine | cloudy | 7 | hazy | 6.5-7 |
| 10 mM sodium phosphate | very cloudy | 7 | cloudy | 5-5.5 |

| Preparation 3 (22 mg/ml ala-TFPI) | | | | |
|---|---|---|---|---|
| in water | clear | 6.5-7 | hazy | 6-6.5 |
| 10 mM L-histidine | clear | 6.5-7 | hazy | 6.5-7 |
| 10 mM sodium phosphate | hazy | 6.5 | cloudy | 5.5-6 |
| 10 mM sodium citrate | cloudy | 6.5 | cloudy | 6 |

To assess if pH had changed at -70°C, pH in the frozen state was measured by a dye color change using the Universal pH solution purchased from Fisher Scientific. The ala-TFPI-polyphosphate formulation was highly sensitive to changes in both the pH and the ionic strength. Ala-TFPI precipitated immediately when formulated in ala-TFPI-polyphosphate formulations either below pH 5.8 or above 80 mM NaCl. Ala-TFPI eventually formed a precipitate at ambient temperature after addition of NaCl or a concentration as low as 5 mM to ala-TFPI-polyphosphate formulations. Addition of more NaCl resulted in a faster precipitation. The sensitivity of ala-TFPI-polyphosphate formulations to changes in pH and salt may explain why ala-TFPI precipitated during a freeze-thaw cycle. The pH of buffers such as citrate and phosphate decreases upon freezing because of partial precipitation of different salt forms that make up the buffer system. A shift in pH or concentration of salts upon freezing can weaken the interaction between ala-TFPI and polyphosphate. The weakened interaction can result in the precipitation of ala-TFPI. Similarly, addition of salt at ambient temperature could also weaken the electrostatic interaction between ala-TFPI and polyphosphate and result in ala-TFPI precipitation.

Among the buffer species tested, L-histidine best stabilized pH upon freezing. In addition, L-histidine also exists as either a simple neutral form or a protonated form in solution. Therefore, it should have a minimal effect on the interaction between ala-TFPI and polyphosphate and thus is a preferred buffer.

### EXAMPLE 5

### Development of Lyophilized Ala-TFPI Formulations

### Initial formulation screen

Screening of formulations for lyophilization used 18 formulations as listed in Table 4. Selection of the formulation components was made on the basis of: 1) solubilizing effects on ala-TFPI of solutes such as L-arginine, citrate, L-histidine, imidazole and polyphosphate; 2) tendency of solutes such as sucrose to form a glass matrix to stabilize lyophilized proteins; and 3) ability of solutes such as mannitol and glycine to form a crystalline cake structure. Most of the formulations have an osmolarity close to isotonic, except formulations 2, 3 and 4, which are approximately twice that of an isotonic solution.

**Table 4**

| Sample number | Formulation | pH | |
|---|---|---|---|
| 1 | 10 mM sodium citrate, 3% (w/v) L-arginine | 6.0 | * |
| 2 | 10 mM sodium citrate, 3% (w/v) L-arginine, 4% (w/v) mannitol | 6.0 | |
| 3 | 10 mM sodium citrate, 3% (w/v) L-arginine, 2% (w/v) glycine | 6.0 | * |
| 4 | 10 mM sodium phosphate, 3% (w/v) L-arginine, 4% (w/v) mannitol | 6.0 | * |
| 5 | 10 mM sodium citrate, 2% (w/v) L-lysine | 6.0 | * |
| 6 | 10 mM sodium citrate, 3% (w/v) L-arginine, 1% (w/v) PEG-400 | 6.0 | * |
| 7 | 100 mM sodium citrate | 6.0 | * |
| 8 | 100 mM sodium citrate | 7.0 | * |
| 9 | 10 mM sodium phosphate, 120 mM sodium sulfate | 6.0 | * |
| 10 | 10 mM sodium citrate, 4.5% (w/v) mannitol, 0.1% (w/v) phosphate glass | 6.0 | * |
| 11 | 10 mM sodium citrate, 8.5% (w/v) sucrose, 0.1% (w/v) phosphate glass | 6.0 | * |
| 12 | 10 mM sodium citrate, 4.5% (w/v) mannitol, 0.1% (w/v) tripolyphosphate | 6.0 | * |
| 13 | 10 mM L-histidine, 4% (w/v) mannitol, 1% (w/v) sucrose | 6.0 | |
| 14 | 10 mM L-histidine, 8.5% (w/v) sucrose | 6.0 | * |
| 15 | 10 mM L-histidine, 2% (w/v) glycine, 1 % (w/v) sucrose | 6.0 | |
| 16 | 10 mM imidazole, 4% (w/v) mannitol, 1 % (w/v) sucrose | 6.5 | |
| 17 | 10 mM imidazole, 2% (w/v) glycine, 1 % (w/v) sucrose | 6.5 | |
| 18 | 10 mM imidazole, 8.5% (w/v) sucrose | 6.5 | * |

| | | | |
|---|---|---|---|
| * for reference | | | |

Visual properties of the lyophilized product were examined after freeze-drying (lyophilizing). Cake morphology was evaluated visually. The results are shown in Table 5. A cake with little shrinkage or collapse was considered to retain "good" cake morphology. Formulations 10, 12, 13, 16 and 17 showed good cake morphology. Of those, four contained mannitol and one contained glycine as the crystal forming agent.

**Table 5**

| Sample number | Cake morphology | Reconstituted Solution Clarity | Residual Moisture (w/w) | Percent Recovery Lyophilization vs. Aqueous | | |
|---|---|---|---|---|---|---|
| | | | | CEX-HPLC | PT assay | |
| 1 | collapsed | clear | 6.31 | 99.7 | 125 | * |
| 2 | shrunken | clear | 2.18 | 94.1 | 126 | |
| 3 | collapsed | clear | 3.70 | 94.6 | 102 | * |
| 4 | shrunken | clear | 2.36 | 93.9 | 105 | * |
| 5 | collapsed | clear | 17.7 | 95.9 | 94 | * |
| 6 | collapsed | clear | 3.36 | 95.8 | 102 | * |
| 7 | collapsed | clear | 7.05 | 94.0 | 81 | * |
| 8 | collapsed | clear | 9.34 | 96.2 | 102 | * |
| 9 | shrunken | slightly turbid | 1.00 | 97.5 | 94 | * |
| 10 | good | slightly turbid | 0.51 | | | * |
| 11 | shrunken | clear | | | | * |
| 12 | good | clear | 0.65 | | | * |
| 13 | good | clear | 0.34 | 92.5 | 86 | |
| 14 | shrunken | clear | | 93.3 | 63.7 | * |
| 15 | shrunken | clear | 0.51 | 100.1 | 107 | |
| 16 | good | clear | | 94.5 | 98.4 | |
| 17 | good | clear | | 99.1 | 97.1 | |
| 18 | shrunken | clear | | 93.3 | 115 | * |

| | | | | | | |
|---|---|---|---|---|---|---|
| * for reference | | | | | | |

Residual moisture is one of the determining factors in preserving proteins in the dried state. Residual moisture of less than 1% (w/w) is preferred. The residual moisture content was determined by the Karl Fischer titration method (Angew. Chemie 48:394 (1935)) for the 13 formulations, and the results are shown in Table 5. Formulations 9, 10, 12, 13 and 15 contained less than 1% (w/w) residual moisture, and formulations 1 through 8 had moisture content greater than 1 % (w/w).

Lyophilized compositions were reconstituted by adding 1 ml "Water For Injection" (WFI), i.e., water which has been approved by the FDA for injection into human patients. Most lyophilized compositions dissolved within 1 minute, and 16 of the 18 reconstituted formulations were clear (Table 5). Reconstituted formulations 9 and 10 were cloudy (Table 5), suggesting that ala-TFPI aggregation occurred in these two formulations during lyophilization.

Reconstituted formulations 1-9 and 13-18 were also analyzed by the PT assay for bioactivity and by CEX-HPLC analysis for loss of soluble ala-TFPI. Results are shown in Table 5. Formulations 10, 11 and 12 were not included in this analysis because the polyphosphate in these formulations interfered with the cation exchange HPLC and the PT assays.

Results from the PT assay showed that all 15 formulations tested were biologically active and indistinguishable from unlyophilized aqueous controls. Therefore, ala-TFPI in these formulations was not particularly sensitive to stress from freeze-drying.

The concentration of soluble ala-TFPI was measured by CEX-HPLC analysis and was less than 10% different from lyophilized samples and aqueous controls. The subtle change in the ala-TFPI concentration was most likely caused by a small volume change during reconstitution. Using formulation 13 as an example, a 5% volume increase occurs when reconstituted with Water For Injection.

### Long term storage stability

The long term storage stability of ten lyophilized compositions (1, 2, 4, 5 and 13-18 of Table 5) was studied. Samples of these formulations were stored at different temperatures and withdrawn at predetermined time intervals for degradation analysis.

First, the degree of aggregation in lyophilized compositions was examined. The loss of soluble protein was measured by CEX-HPLC analysis. Results obtained for samples stored up to 3 months at either 40°C or 50°C are shown in Table 6. Ala-TFPI was quite stable in these formulations. Bioactivity in the lyophilized compositions was determined by the PT assay and is shown in Table 6. Samples stored up to 3 months at either 40 °C or 50 °C retained about 40-100% of their initial bioactivity upon reconstitution (Table 6).

**Table 6**

| Incubation | Percent of Initial Ala-TFPI for Formulation Number | | | | | | | | | |
|---|---|---|---|---|---|---|---|---|---|---|
| Time | 1* | 2 | 4* | 5* | 13 | 14* | 15 | 16 | 17 | 18* |
| *50 °C incubation* | | | | | | | | | | |
| 1 wk | 99.6 | 102 | 99.9 | 97.9 | 100 | 77.8 | 82.6 | 88.4 | 95.9 | 92.2 |
| 2 wk | 101 | 99.6 | 96.4 | 95.8 | 97.9 | 74.1 | 95.0 | 94.8 | 96.6 | 89.9 |
| 1 mo | 105 | 96.4 | 95.3 | 98.0 | 98.2 | 76.0 | 97.8 | 98.1 | 97.7 | 26.2 |
| 3 mo | 96.3 | 87.0 | 86.1 | | 92.1 | 68.2 | 90.9 | 44.2 | 99.4 | 37.6 |

| *40°C incubation* | | | | | | | | | | |
|---|---|---|---|---|---|---|---|---|---|---|
| 2 wk | 105 | 93.4 | 98.2 | 97.0 | 84.5 | 69.3 | 88.6 | 79.4 | 99.2 | 93.6 |
| 1 mo | 103 | 93.4 | 97.7 | 98.8 | 96.1 | 75.9 | 96.9 | 96.1 | 100 | 97.8 |
| 3 mo | 98.2 | 90.9 | 90.7 | | 91.0 | 72.8 | 92.2 | 93.9 | 94.4 | 94.5 |

| B (PT Assay) | | | | | | | | | | |
|---|---|---|---|---|---|---|---|---|---|---|
| Incubation | Percent of Initial Ala-TFPI for Formulation Number | | | | | | | | | |
| Time | 1 * | 2 | 4 * | 5* | 13 | 14* | 15 | 16 | 17 | 18* |
| *50 °C incubation* | | | | | | | | | | |
| 1 wk | 99.2 | 93.1 | 89.3 | 101 | 88.1 | 123 | 68.4 | 77.9 | 70.7 | 57.8 |
| 2 wk | 92.9 | 93.9 | 84.9 | 104 | 101 | 146 | 91 | 114 | 116 | 100 |
| 1 mo | 93.3 | 78.0 | 78.1 | 88.1 | 91.3 | 123 | 85.8 | 93.1 | 83.8 | 32.8 |
| 3 mo | 78.6 | 73.9 | 69.1 | 74.5 | 75.4 | 99.1 | 64.1 | 62.3 | 59.1 | 43.2 |

| *40°C incubation* | | | | | | | | | | |
|---|---|---|---|---|---|---|---|---|---|---|
| 2 wk | 97.0 | 85.6 | 81.5 | 93.5 | 99.9 | 123 | 90.9 | 62.2 | 61.7 | 59.3 |
| 1 mo | 60.1 | 80.9 | 80.1 | 83.1 | 86.6 | 121 | 82.2 | 87.5 | 89.4 | 71.8 |
| 3 mo | 77.9 | 73.3 | 72.6 | 75.7 | 70.2 | 94.3 | 63.3 | 76.8 | 66.8 | 51.3 |

| | | | | | | | | | | |
|---|---|---|---|---|---|---|---|---|---|---|
| * see Table 4 for definition of formulation * for reference | | | | | | | | | | |

Formulation 13 was selected for analysis by cation exchange HPLC, reverse phase HPLC, and size exclusion HPLC as described above. FIG. 6 shows chromatograms of CEX-HPLC, RP-HPLC and SEC-HPLC for samples of this formulation stored at different temperatures for 3 months.

Ala-TFPI eluted from the CEX-HPLC as a single peak with a retention time at about 18 min. This single peak profile on the CEX-HPLC chromatograms remained unchanged for all storage temperatures for this formulation (FIG. 6A).

The elution RP-HPLC profile of ala-TFPI was quite complex. A main ala-TFPI species eluted at 19 min, two oxidized ala-TFPI species eluted slightly faster, and several minor acetylated TFPI species eluted slower. As shown in FIG. 6B, this elution profil2e was also observed for samples of lyophilized ala-TFPI that were stored for 3 months at various temperatures.

Size exclusion-HPLC elution profiles for lyophilized ala-TFPI samples stored at various temperatures were essentially unchanged after storage. There were no changes detected for the monomer species eluting at 8.25 minutes and the buffer species eluting around 11 minutes. The sample at 50°C showed some changes in the buffer peaks (FIG. 6C), but this was probably caused by the oxidation of the histidine component at the elevated temperature.

Although accelerated stability tests showed a difference among the formulations, real time stability examination of the different compositions stored for 6 months at 2-8°C showed no detectable changes by cation exchange HPLC analysis. FIG. 7 shows chromatograms of CEX-HPLC for samples of five formulations after storage for 6 months at either 2-8°C or 50°C. Although extensive degradation was observed in some of the samples stored at 50°C (e.g., formulations 4 and 17), no change was detected in the five samples stored at 2-8°C.

Aggregation of ala-TFPI resulted in visible precipitates that could be filtered out using a 0.22 µm filter. Thus, a decrease in the integrated area of the original peaks was observed on HPLC chromatograms. No significant changes other than the aggregation were detected on these HPLC chromatograms. Similarly, no degraded species were observed on SDS-PAGE gels. Therefore, aggregation was the major degradation pathway for this protein in the freeze-dried state.

### EXAMPLE 6

### Effect of buffer species, pH and Ala-TFPI concentration on stability of lyophilized Ala-TFPI

The effect of buffer species, pH, and ala-TFPI concentration on the stability of lyophilized ala-TFPI compositions was investigated. Buffer species, including L-histidine, citrate and imidazole, were compared at pH 6.5 in a formulation containing 10 mM buffer and 4% (w/v) mannitol. Table 7 shows loss of soluble ala-TFPI measured by CEX-HPLC analysis for samples stored for 5 weeks at either 40°C or 50°C. As shown in Table 7, L-histidine was the best buffer species for preserving soluble ala-TFPI, followed by citrate. For example, the fraction of the soluble ala-TFPI still remaining after a 5-week storage at 50°C was 95%, 41%, and 10% for L-histidine, citrate, and imidazole, respectively. This correlates with the order of their tendency to form glass under the specific freeze-drying condition employed in this study.

**Table 7**

| Formulation | Percent of Initial Ala-TFPI Concentration by CEX-HPLC | |
|---|---|---|
| | 50°C | 40 °C |
| 10 mM L-histidine, 4% (w/v) mannitol, 1 % (w/v) sucrose, pH 5.5* | 105 | |
| 10 mM L-histidine, 4% (w/v) mannitol, 1 % (w/v) sucrose, pH 6.0 | 101 | |
| 10 mM L-histidine, 4% (w/v) mannitol, 1 % (w/v) sucrose, pH 6.5* | 95.9 | 95.4 |
| 10 mM L-histidine, 4% (w/v) mannitol, pH 6.0* | 98.4 | |
| 10 mM L-histidine, 4% (w/v) mannitol, pH 6.5- | 94.7 | 93.8 |
| 10 mM sodium citrate, 4% (w/v) mannitol, pH 6.5* | 41.1 | 50.0 |
| 10 mM imidazole, 4% (w/v) mannitol; pH 6.5* | 10.2 | 45.5 |

| | | |
|---|---|---|
| * for reference | | |

In a separate experiment, the effects of citrate and phosphate were compared in two formulations containing 10 mM buffer at pH 6.0, 3% (w/v) L-arginine and 4% (w/v) mannitol. Citrate was better than phosphate in preserving ala-TFPI stability (FIG. 7).

The effect of pH on the stability of lyophilized ala-TFPI was tested using formulation 13 (Table 4). The pH range examined was a narrow range from about pH 5.5 to about pH 6.5. The results of CEX-HPLC analysis and the PT assay are shown in Table 7. There was a small difference in stability for the range of pH values tested. The lower pH value, pH 5.5, resulted in higher stability of lyophilized ala-TFPI than pH 6.0 or pH 6.5.

Stability of ala-TFPI in formulation 13 was investigated at protein concentrations ranging from 100µg/ml to 1,500 µg/ml. Table 8 shows the difference in protein concentration between samples stored at 50°C and samples stored at 2-8°C. Samples containing higher TFPI concentration were slightly more stable than those having lower ala-TFPI concentrations and exhibited a lower loss of ala-TFPI at 50°C. Reduced stability became noticeable only when the ala-TFPI concentration was below 250 µg/ml.

**Table 8**

| Ala- TFPI conc. (µg/ml) | Percent Difference 50°C storage vs. 2-8°C storage |
|---|---|
| 50 | 93.6 |
| 150 | 98.0 |
| 250 | 103.5 |
| 500 | 101.1 |
| 1000 | 100.3 |

### EXAMPLE 7 (reference example)

### Stabilizing Effect of Polyphosphate on Lyophilized Compositions of Alα-TFPI

In these studies, ala-TFPI to polyphosphate weight ratios of 2:1, 6:1 and 8:1 were tested for the ability of polyphosphate to stabilize lyophilized compositions of TFPI. Buffers such as 10 mM histidine and 10 mM imidazole were added to the formulations to maintain the pH at 7. Mannitol and sucrose were added to certain formulations to increase cake strength and to enhance the stability, respectively. The ala-TFPI concentration was adjusted to 20 mg/ml in the formulations prior to lyophilization.

Results of residual moisture content and cake morphology are shown in Table 9. All six formulations show less than a half percent residual moisture. Polyphosphate itself seems to be a good agent for lyophilization and gives a slightly shrunken cake without any other additives. With the addition of histidine, no further improvement on the cake morphology was observed. However, addition of mannitol and sucrose in the formulation improved the cake morphology. In a later experiment, freeze-drying of an ala-TFPI-polyphosphate formulation at 2:1 weight ratio with 4% (w/v) mannitol alone also yielded good cake structure. Therefore, mannitol was found to be a good crystal forming agent for product elegance.

**Table 9**

| Ala-TFPI/Polyphosphate ratio | Formulation | Residual Moisture | Cake Morphology |
|---|---|---|---|
| 6:1 | water | 0.44% | slightly shrunken |
| 6:1 | 10 mM histidine | 0.19% | slightly shrunken |
| 6:1 | 10 mM histidine, 4% (w/v) mannitol, 1% (w/v) sucrose | 0.34% | good cake |
| 6:1 | 10 mM imidazole, 4% (w/v) mannitol, 1% (w/v) sucrose | 0.16% | good cake |
| 2:1 | 10 mM histidine, 4% (w/v) mannitol, 1% (w/v) sucrose | 0.27% | good cake |
| 8:1 | 10 mM histidine, 4% (w/v) mannitol, 1% (w/v) sucrose | 0.22% | good cake |

Accelerated stability tests were carried out at 40°C and 50°C. Typical RP-HPLC chromatograms of the samples tested are shown in FIG. 8. Similar to the aqueous formulations, loss of ala-TFPI due to aggregation was observed during storage at elevated temperatures and resulted in a decrease in peak area on the chromatograms.

Results of RP-HPLC analysis showing loss of ala-TFPI in the samples tested are shown in Table 10. All formulations shown in Table 10 were more stable than the aqueous high concentration formulation alone. After 3 months storage at 40°C, the aqueous formulation samples contained no more than 50% soluble TFPI by RP-HPLC (Table 2), while all lyophilized formulations contained more than 80% soluble ala-TFPI.

**Table 10**

| Ala-TFPI/ Polyphosphate Ratio | Formulation (all at pH 7.0) | Percent Soluble Ala-TFPI Remaining | | | |
|---|---|---|---|---|---|
| | | 40°C | | 50°C | |
| | | 1 mo | 3 mo | 1 mo | 3 mo |
| 6:1 | water | 93.9 | 88.3 | 83.9 | 1.3 |
| 6:1 | 10 mM histidine | 95.5 | 94.0 | 70.6 | <1 |
| 6:1 | 10 mM histidine, 4% (w/v) mannitol, 1% (w/v) sucrose | 93.5 | 92.8 | N/A | <1 |
| 6:1 | 10 mM Imidazole, 4% (w/v) mannitol, 1% (w/v) sucrose | 95.5 | 93.3 | N/A | <1 |
| 2:1 | 10 mM histidine, 4% (w/v) mannitol, 1% (w/v) sucrose | 96.7 | 95.9 | 82.7 | 56.3 |
| 8:1 | 10 mM histidine, 4% (w/v) mannitol, 1% (w/v) sucrose | 99.4 | 95.4 | 92.8 | 1.1 |

Among the six formulations shown in Table 10, the formulation with an ala-TFPI to polyphosphate ratio of 2:1 showed the best ala-TFPI stability. After 3 months storage at 50°C this formulation still contained 56% soluble ala-TFPI by RP-HPLC, whereas other formulations were almost entirely degraded. The degradation kinetics of this formulation at 50°C compared with the high concentration formulation are shown in FIG. 9. The lyophilized formulation is approximately 10 times more stable than the aqueous formulation as examined in this accelerated stability test.

The differential scanning calorimetry (DSC) measurement revealed that the glass transition temperature (Tg) for polyphosphate in the histidine/mannitol/sucrose formulation was about -28°C. Because the product temperature was below -28°C during the first 11 hr of primary drying as shown in FIG. 10, polyphosphate probably formed glass upon freeze-drying. Therefore, the stabilization of polyphosphate provided to ala-TFPI in lyophilized form can still follow the glass stabilization theory, which states that the rigid glass greatly reduces diffusion of molecules to eliminate degradation reactions.

Because sucrose also is a good glass former with a Tg of about -32°C, it was of interest to test if polyphosphate alone could stabilize ala-TFPI by forming glass. In a separate experiment, two formulations containing 20 mg/ml ala-TFPI, 10 mg/ml polyphosphate, 10 mM L-histidine at pH 7, and 4% mannitol with or without 1% sucrose were prepared and lyophilized. Both formulations showed similar cake morphology and low residual moisture levels (0.69% without 1% sucrose and 0.49% with 1% sucrose). Sucrose seems to have little effect on TFPI during freeze-drying.

The stability of these two formulations was compared by examining the decrease in both the protein concentration by the RP-HPLC and the *in vitro* specific activity by the PT assay upon storage at elevated temperatures. As shown in Table 11, the difference in stability for these two formulations is insignificant. Both formulations showed 2% loss in ala-TFPI after being stored at 60°C for two weeks. At 50°C for 4 weeks, formulations with and without sucrose showed an ala-TFPI loss of 12% and 18%, respectively. Additionally, the specific activities of these two formulations at 50°C for 4 weeks were comparable.

**Table 11**

| Formulation | 2 weeks at 60°C | | 4 weeks at 50°C | |
|---|---|---|---|---|
| | Percent Ala-TFPI Remaining | Specific Activity | Percent Ala-TFPI Remaining | Specific Activity |
| with sucrose | 98% | 0.93 | 88% | 0.96 |
| without sucrose | 98% | 0.37 | 82% | 0.90 |

### EXAMPLE 8

### Preparing Ala-TFPI Formulations for Lyophilization

Manufacturing of an ala-TFPI formulation frequently requires a buffer exchange process. Bulk ala-TFPI may contain, for example, 10 mg/ml protein, 2M urea, 20 mM sodium phosphate at pH 7.2 and 150 mM NaCl. Lyophilized formulations do not generally contain urea or NaCl. To process ala-TFPI from the urea-containing bulk into a formulation buffer (Table 4), urea and NaCl have to be removed by a buffer exchange method such as dialysis, gel filtration, or diafiltration. Among these methods, diafiltration is preferred from a manufacturing view point because of the large volumes of ala-TFPI involved in manufacturing.

### One-step diafiltration

Diafiltration of ala-TFPI bulk containing, for example, 10 mg/ml protein, 2M urea, 20 mM sodium phosphate at pH 7.2, and 150 mM NaCl directly into several pH 6 or 6.5 formulation buffers resulted in extensive protein precipitation. As shown in Table 12, diafiltration into four buffers at either pH 6 or 6.5 resulted in either a turbid solution or a lower yield. However, diafiltration into a pH 4 buffer containing 10 mM L-glutamate, 4% (w/v) mannitol and 1% (w/v) sucrose resulted in a clear solution with a high yield.

**Table 12**

| Diafiltration Buffer | pH | Clarity of Diafiltrate | Percent Recovery of Soluble Ala-TFPI |
|---|---|---|---|
| 10mM L-glutamate, 4% (w/v) mannitol, 1% (w/v) sucrose* | 4.0 | clear | 95 |
| 10mM L-histidine, 4% (w/v) mannitol, 3% (w/v) L-arginine* | 6.0 | clear | 85 |
| 10mM L-histidine, 2% (w/v) glycine, 1% (w/v) sucrose | 6.0 | turbid | 89 |
| 10mM imidazole, 4% (w/v) mannitol, 1% (w/v) sucrose | 6.5 | turbid | 81 |
| 10mM imidazole, 2% (w/v) glycine, 1% (w/v) sucrose | 6.5 | turbid | 87 |

| | | | |
|---|---|---|---|
| * for reference | | | |

The ala-TFPI concentration used in the diafiltration (1 mg/ml) was much lower than the solubility limit in both the starting buffer and the final formulation buffers (5-10 mg/ml solubility). These two buffer systems apparently solubilize ala-TFPI by two different mechanisms. The starting buffer contains urea and salt and is relatively high in ionic strength, while some of the formulation buffers are low in ionic strength. Diafiltration is a relatively slow process. Urea and salt in the bulk buffer are gradually diafiltered out, and formulation buffer components are gradually diafiltered in. During diafiltration, ala-TFPI can experience transient solvent conditions which affect either ala-TFPI solubility or ala-TFPI stability.

We examined turbidity change of the ala-TFPI formulation in the diafiltration vessel at each volume of buffer exchange. The starting formulation was 1 to 1.5 mg/ml TFPI in 1 M urea, 10 mM sodium phosphate at pH 7 and 125 mM NaCl. Results are shown in Table 13.

**Table 13**

| Diafiltration buffer | ¹ Solution clarity observed during diafiltration at buffer exchange volume | | | | | | | | | | |
|---|---|---|---|---|---|---|---|---|---|---|---|
| | 0 | 1 | 2 | 3 | 4 | 5 | 6 | 7 | 8 | 9 | 10 |
| 10 mM Na acetate, pH 4.0 * | - | + | - | - | - | - | - | - | | | |
| 10 mM succinic acid, pH 4.0 * | + | + | - | - | - | - | - | - | | | |
| 10 mM Na citrate, pH 4.0 * | - | - | - | - | - | - | - | - | | | |
| 10 mM L-glutamate, 4% (w/v) mannitol, 1% (w/v) sucrose, pH 4.0 * | - | + | - | - | - | - | - | - | - | - | - |
| 10 mM Na acetate, pH 5.5 * | - | + | + | - | - | + | - | - | | | |
| 10 mM L-histidine, 2% (w/v) glycine, 1% (w/v) sucrose, pH 6.0 * | - | + | + | - | - | + | ++ | ++ | | | |
| 10 mM L-histidine, 4% (w/v) mannitol, 3% (w/v) L-arginine, pH 6.0 * | - | - | - | - | - | - | - | - | - | - | |
| 10 mM imidazole, 4% (w/v) mannitol, 1% (w/v) sucrose, pH 6.5 | - | + | + | - | - | - | ++ | ++ | | | |
| 10 mM imidazole, 2% (w/v) glycine, 1% (w/v) sucrose, pH 6.5 | - | + | + | - | - | + | ++ | ++ | | | |
| 10 mM imidazole, pH 6.5 * | + | + | - | - | - | - | - | + | | | |
| 10 mM L-histidine, pH 6.5 * | + | + | - | - | + | + | + | + | | | |

| | | | | | | | | | | | |
|---|---|---|---|---|---|---|---|---|---|---|---|
| ¹ "+" indicates a slightly turbid solution, "++" indicates a cloudy solution and "-" indicates a clear solution. * for reference | | | | | | | | | | | |

Ala-TFPI diafiltered at pH 4 showed a transient change in turbidity at the first or second volume of buffer exchange; it then became clear and remained so all the way to the end of diafiltration. Using a sodium acetate pH 5.5 buffer, turbidity change was observed at 1, 2 and 5 volumes of buffer exchange. Diafiltration using pH 6 or pH 6.5 resulted in turbid solutions at the late stage of diafiltration, except for a solution containing 3% (w/v) L-arginine. Therefore, both low pH and L-arginine prevented ala-TFPI from precipitation during diafiltration.

### Two-step diafiltration

Precipitation of ala-TFPI during diafiltration can be prevented by the use of buffers having a pH of about 4. To test if a buffer having a pH of about 4 could serve as a bridge between the bulk buffer and the final formulation buffer, a two-step diafiltration experiment was carried out. At the first step, TFPI was diafiltered against a pH 4 buffer for four volumes of buffer exchange. At the second step, the diafiltration buffer was switched to the formulation buffer, and six volumes of buffer exchange were performed. The turbidity changes during diafiltration for several two-step diafiltration buffer systems were recorded and are shown in Table 14.

The pH 4 buffer preferably is an L-glutamate buffer. After four volume changes with the glutamate buffer, the desired final product buffer with a suitable solubilizing agent is introduced as the second step. This process yields a clear, formulated final product with much less loss of protein incurred during processing.

In one experiment, the starting solution was ala-TFPI at 1 mg/ml in 1 M urea, 10 mM sodium phosphate, 125 mM NaCl, pH 7, was first diafiltered by a L-glutamate buffer (10 mM L-glutamic acid/L-glutamate, 4% (w/v) mannitol, 1% (w/v) sucrose at pH 4.0) for four volumes change (step 1), and then by an L-histidine or imidazole buffer for additional six volumes change (step 2). clarity of the diafiltered solution was recorded. The diafiltered solution was filtered through a 0.22 µm filter unit, and ala-TFPI concentration of the filtered solution was measured by UV absorbance and compared with that before diafiltration to calculate the recovery. The results are shown in Table B.

**Table B. (for reference)**

| Diafiltration buffer at step 2 | Clarity of diafiltrate | % recovery of soluble ala-TFPI |
|---|---|---|
| 10 mM L-histidine, 4% mannitol, 1% sucrose | clear | 88 |
| 10 mM imidazole, 4% mannitol, 1% sucrose | clear | 83 |

This procedure can be used for preparation of liquid (or, solution) formulations of TFPI or TFPI variant for therapeutic use at an appropriate commercial scale.

**Table 14**

| Diafiltration buffer | ‡ Solution clarity observed during diafiltration at buffer exchange volume | | | | | | | | | | |
|---|---|---|---|---|---|---|---|---|---|---|---|
| | 0 | 1 | 2 | 3 | 4 | 5 | 6 | 7 | 8 | 9 | 10 |
| 10 mM L-arginine, 150 mM NaCl, pH 4.0 * | - | - | - | - | - | | | | | | |
| 10 mM imidazole, 4% (w/v) mannitol, 1% (w/v) sucrose, pH 6.5 | | | | | | + | ++ | ++ | ++ | | |
| 10 mM L-glutamine, 4% (w/v) mannitol, 1% (w/v) sucrose, pH 4.0 * | - | + | - | - | - | | | | | | |
| 10 mM L-histidine, 4% (w/v) mannitol, 1% (w/v) sucrose, pH 6.0 | | | | | | - | - | - | - | - | - |
| 10 mM L-glutamine, 4% (w/v) mannitol, 1% (w/v) sucrose, pH 4.0 * | - | + | - | - | - | | | | | | |
| 10 mM imidazole, 4% (w/v) mannitol, 1% (w/v) sucrose, pH 6.5 | | | | | | - | - | - | - | - | - |

| | | | | | | | | | | | |
|---|---|---|---|---|---|---|---|---|---|---|---|
| ‡ "+" indicates a slightly turbid solution, "++" indicates a cloudy solution and "-" indicates a clear solution. * for reference | | | | | | | | | | | |

As shown in Table 14, if the first step diafiltration used a relatively high ionic strength buffer, such as 10 mM L-glutamate at pH 4 and 140 mM NaCl, no transient turbidity change was observed during the diafiltration. However, ala-TFPI precipitated at the second step diafiltration, which used a low ionic strength buffer. On the other hand, if the first step diafiltration used a low ionic strength buffer, such as 10 mM L-glutamate at pH 4, 4% (w/v) mannitol and 1% (w/v) sucrose, ala-TFPI solution passed a short transient turbid stage at about 1 volume of buffer exchange, and then no turbidity change was detected thereafter during diafiltration. Thus, using a low ionic strength pH 4 buffer, the two-step diafiltration process yields a clear formulated final product.

### EXAMPLE 9 (reference example)

### 50°C accelerated stability assay

Accelerated stability testing was used to determine the stability of various concentrations of ala-TFPI. Samples were prepared in 20 mM sodium citrate at pH 5.5 and 300 mM arginine. Ala-TFPI concentrations ranged from about 1 mg/ml to about 7.4 mg/ml. The aqueous samples were incubated at 50°C for up to about six months. Each month, the percent remaining soluble ala-TFPI was determined for each concentration. Soluble ala-TFPI was determined by cation exchange HPLC, and the results are shown in FIG. 11. As shown in FIG. 11, ala-TFPI is slightly more stable at concentrations below about 4 mg/ml.

### EXAMPLE 10 (reference example)

### Survival studies

A murine cecal ligation and puncture study was conducted to compare a freshly prepared, clinical grade lot of recombinant ala-TFPI (rTFPI) (TFPI 92) with clinical grade material that was partially deamidated and oxidized (TFPI 78). This model induces a polymicrobial intraperitoneal and systemic infection by direct fecal contamination and cecal necrosis, closely mimicking human intra-abdominal sepsis. Opal et al., Critical Care Medicine 29, 13-18, 2001.

Both preparations of TFPI were prepared as described in Serial No. 60/494,546 filed August 13, 2003, Serial No. 60/60/509,277 filed October 8, 2003, and Serial No. 60/512,199 filed October 20, 2003; Either rTFPI 78, rTFPI 92 or diluent control was given in a blinded fashion over 48 hours (SQ q12 hours x four doses). Prior to and 48 hours after the surgical procedure, blood was drawn to determine the level of quantitative bacteremia, endotoxin and cytokines (tumor necrosis factor-alpha and interleukin-6). The animals were observed daily and deaths were recorded as they occurred. All animals underwent necropsy evaluation for histological evidence of organ injury and quantitative bacteriology at the end of the experimental period.

The Kaplan-Meier survival plots are depicted in FIG. 12. There was a significant survival advantage for the mice who received the freshly prepared rTFPI as compared with the partially oxidized, deamidated form of rTFPI. Both rTFPI groups fared better than those mice that received diluent in the control group. As expected the sham-operated mice (surgical intervention with identification of the cecum but no ligation and puncture) survived the seven day study period. There were no significant differences in the secondary endpoints of bacteremia, endotoxemia, or cytokine production between the two rTFPI-treated groups.

This study demonstrates that TFPI seems to offer a survival advantage through a mechanism not explained by blood levels of bacteria, endotoxin, or cytokines. Deamidated, oxidized TFPI offered less protection than freshly prepared TFPI.

## Claims

1. A lyophilized composition comprising TFPI or ala-TFPI wherein before lyophilization the TFPI or ala-TFPI is present in an aqueous formulation selected from the group of formulations consisting of:
% (w/v) arginine, 4% (w/v) mannitol, and 10 mM sodium citrate, with a pH of 6;
3% (w/v) arginine, 2% (w/v) glycine, and 10 mM sodium citrate, with a pH of 6;
1% (w/v) sucrose, 4% (w/v) mannitol, and 10 mM L-histidine, with a pH of 6;
1% (w/v) sucrose, 2% (w/v) glycine, and 10 mM histidine, with a pH of 6;
1% (w/v) sucrose, 4% (w/v) mannitol, and 10 mM imidazole, with a pH of 6.5; and
1% (w/v) sucrose, 2% (w/v) glycine, and 10 mM imidazole, with a pH of 6.5.

2. The lyophilized composition as claimed in claim 1 wherein the aqueous formulation comprises a concentration of TFPI or ala-TFPI selected from the group of concentrations consisting of:
no more than 10 mg/ml of the TFPI or ala-TFPI;
no more than 1 mg/ml of the TFPI or ala-TFPI; and
no more than 0.2 mg/ml of the TFPI or ala-TFPI.

3. The lyophilized composition as claimed in claim 1 or claim 2 comprising ala-TFPI.

## Patentansprüche

1. Lyophilisierte Zusammensetzung, umfassend TFPI oder Ala-TFPI, wobei das TFPI oder Ala-TFPI vor der Lyophilisierung in einer wässrigen Formulierung vorliegt, ausgewählt aus der Gruppe von Formulierungen bestehend aus:
3% (Gew.Nol.) Arginin, 4% (Gew./Vol.) Mannit und 10 mM Natriumcitrat mit einem ph-Wert von 6;
3% (Gew./Vol.) Arginin, 2% (Gew.Nol.) Glycin und 10 mM Natriumcitrat mit einem ph-Wert von 6;
1% (Gew.Nol.) Saccharose, 4% (Gew.Nol.) Mannit und 10 mM L-Histidin mit einem ph-Wert von 6;
1% (Gew./Vol.) Saccharose, 2% (Gew.Nol.) Glycin und 10 mM Histidin mit einem ph-Wert von 6;
1% (Gew.Nol.) Saccharose, 4% (Gew.Nol.) Mannit und 10 mM Imidazol mit einem ph-Wert von 6,5; und
1% (Gew./Vol.) Saccharose, 2% (Gew.Nol.) Glycin und 10 mM Imidazol mit einem ph-Wert von 6,5.

2. Lyophilisierte Zusammensetzung wie in Anspruch 1 beansprucht, wobei die wässrige Formulierung eine Konzentration an TFPI oder Ala-TFPI umfasst, die ausgewählt ist aus der Gruppe von Konzentrationen bestehend aus:
nicht mehr als 10 mg/ml des TFPI oder Ala-TFPI;
nicht mehr als 1 mg/ml des TFPI oder Ala-TFPI; und
nicht mehr als 0,2 mg/ml des TFPI oder Ala-TFPI.

3. Lyophilisierte Zusammensetzung wie in Anspruch 1 oder 2 beansprucht, umfassend Ala-TFPI.

## Revendications

1. Composition lyophilisée comprenant un TFPI ou ala-TFPI dans laquelle, avant lyophilisation, le TFPI ou ala-TFPI est présent dans une formulation aqueuse choisie dans le groupe de formulations consistant en :
3 % (poids/volume) d'arginine, 4 % (p/v) de mannitol et du citrate de sodium 10 mM à un pH de 6 ;
3% (p/v) d'arginine, 2% (p/v) de glycine et du citrate de sodium 10 mM à un pH de 6 ;
1 % (p/v) de saccharose, 4 % (p/v) de mannitol et de la L-histidine 10 mM à un pH de 6 ;
1 % (p/v) de saccharose, 2 % (p/v) de glycine et de l'histidine 10 mM à un pH de 6 ;
1 % (p/v) de saccharose, 4 % (p/v) de mannitol et de l'imidazole 10 mM à un pH de 6,5 ; et
1 % (p/v) de saccharose, 2 % (p/v) de glycine et de l'imidazole 10 mM à un pH de 6,5.

2. Composition lyophilisée selon la revendication 1, dans laquelle la formulation aqueuse comprend une concentration de TFPI ou d'ala-TFPI choisie dans le groupe de concentrations consistant en :
pas plus de 10 mg/ml de TFPI ou d'ala-TFPI ;
pas plus de 1 mg/ml de TFPI ou d'ala-TFPI ; et
pas plus de 0,2 mg/ml de TFPI ou d'ala-TFPI.

3. Composition lyophilisée selon la revendication 1 ou la revendication 2 comprenant le ala-TFPI.


### SEQUENCE LISTING

<110> Chiron Corporation
<120> Stabilized Lyophilized Compositions Comprising Tissue Factor Pathway Inhibitor or Tissue Factor Pathway Inhibitor Variants
<130> 012441.00053
<150> US 60/438,524
   <151> 2003-01-08
<150> US 60/494,547
   <151> 2003-08-13
<150> US 60/509,276
   <151> 2003-10-08
<150> US 60/512,092
   <151> 2003-10-20
<160> 1
<170> FastSEQ for Windows Version 4.0
<210> 1
   <211> 276
   <212> PRT
   <213> Homo sapiens
   <400> 1
   Asp Ser Glu Glu Asp Glu Glu His Thr Ile Ile Thr Asp Thr Glu Leu
   1 5 10 15
   Pro Pro Leu Lys Leu Met His Ser Phe Cys Ala Phe Lys Ala Asp Asp
   20 25 30
   Gly Pro Cys Lys Ala Ile Met Lys Arg Phe Phe Phe Asn Ile Phe Thr
   35 40 45
   Arg Gln Cys Glu Glu Phe Ile Tyr Gly Gly Cys Glu Gly Asn Gln Asn
   50 55 60
   Arg Phe Glu Ser Leu Glu Glu Cys Lys Lys Met Cys Thr Arg Asp Asn
   65 70 75 80
   Ala Asn Arg Ile Ile Lys Thr Thr Leu Gln Gln Glu Lys Pro Asp Phe
   85 90 95
   Cys Phe Leu Glu Glu Asp Pro Gly Ile Cys Arg Gly Tyr Ile Thr Arg
   100 105 110
   Tyr Phe Tyr Asn Asn Gln Thr Lys Gln Cys Glu Arg Phe Lys Tyr Gly
   115 120 125
   Gly Cys Leu Gly Asn Met Asn Asn Phe Glu Thr Leu Glu Glu Cys Lys
   130 135 140
   Asn Ile Cys Glu Asp Gly Pro Asn Gly Phe Gln Val Asp Asn Tyr Gly
   145 150 155 160
   Thr Gln Leu Asn Ala Val Asn Asn Ser Leu Thr Pro Gln Ser Thr Lys
   165 170 175
   Val Pro Ser Leu Phe Glu Phe His Gly Pro Ser Trp Cys Leu Thr Pro
   180 185 190
   Ala Asp Arg Gly Leu Cys Arg Ala Asn Glu Asn Arg Phe Tyr Tyr Asn
   195 200 205
   Ser Val Ile Gly Lys Cys Arg Pro Phe Lys Tyr Ser Gly Cys Gly Gly
   210 215 220
   Asn Glu Asn Asn Phe Thr Ser Lys Gln Glu Cys Leu Arg Ala Cys Lys
   225 230 235 240
   Lys Gly Phe Ile Gln Arg Ile Ser Lys Gly Gly Leu Ile Lys Thr Lys
   245 250 255
   Arg Lys Arg Lys Lys Gln Arg Val Lys Ile Ala Tyr Glu Glu Ile Phe
   260 265 270
   Val Lys Asn Met
   275